# EUROPEAN PATENT SPECIFICATION

(11) **EP 1 363 663 B1**
(45) Date of publication and mention of the grant of the patent: **02.03.2011**
(21) Application number: 02733782.3
(22) Date of filing: 14.01.2002
(51) Int. Cl.: A61K 39/21, A61K 39/00, A61P 31/18

(54) **NUCLEIC ACID MUCOSAL IMMUNIZATION**
NUKLEINSÄURE MUKOSALE IMMUNISIERUNG
IMMUNISATION DES MUQUEUSES PAR ACIDE NUCLEIQUE

(30) Priority: 12.01.2001 US 261554 P; 27.11.2001 US 333861 P
(43) Date of publication of application: 26.11.2003
(60) Divisional application: 10003888.4
(73) Proprietor: Novartis Vaccines and Diagnostics, Inc., Emeryville, CA 94608 (US)
(72) Inventor: VAJDY, Michael, Emeryville, CA 94608 (US); POLO, John, Emeryville, CA 94608 (US); DUBENSKY, Thomas, Jr., Piedmont, CA 94611 (US); O'HAGAN, Derek, Berkeley, CA 94704 (US)
(74) Representative: Hallybone, Huw George
(86) International application number: PCT/US2002/001031
(87) International publication number: WO 2002/080982

(56) References cited:
- US-A- 6 015 686
- BELYAKOV IGOR M ET AL: "Induction of a mucosal cytotoxic T-lymphocyte response by intrarectal immunization with a replication-deficient recombinant vaccinia virus expressing human immunodeficiency virus 89.6 envelope protein." JOURNAL OF VIROLOGY, vol. 72, no. 10, October 1998 (1998-10), pages 8264-8272, XP002212653 ISSN: 0022-538X
- KANO M. ET AL: "Induction of SIV-specific immune responses by using recombinant sendai viral vector" 7TH CONFERENCE ON RETROVIRUSES AND OPPORTUNISTIC INFECTIONS, [Online] 30 January 2000 (2000-01-30) - 2 February 2000 (2000-02-02), XP002212654 Retrieved from the Internet: <URL:http://www.retroconference.org/2000/a bstracts/807.htm> [retrieved on 2002-09-05]
- VILLACRES MARIA C ET AL: "Maintenance of CD8+ T-cell memory following infection with recombinant Sindbis and vaccinia viruses." VIROLOGY, vol. 270, no. 1, 25 April 2000 (2000-04-25), pages 54-64, XP002212655 ISSN: 0042-6822
- CALEY IAN J ET AL: "Humoral, mucosal, and cellular immunity in response to a human immunodeficiency virus type 1 immunogen expressed by a Venezuelan equine encephalitis virus vaccine vector." JOURNAL OF VIROLOGY, vol. 71, no. 4, 1997, pages 3031-3038, XP002212656 ISSN: 0022-538X
- DATABASE BIOSIS [Online] BIOSCIENCES INFORMATION SERVICE, PHILADELPHIA, PA, US; 2001 VAJDY MICHAEL ET AL: "Human immunodeficiency virus type 1 Gag-specific vaginal immunity and protection after local immunizations with Sindbis virus-based replicon particles." Database accession no. PREV200200304040 XP002212657 cited in the application & JOURNAL OF INFECTIOUS DISEASES, vol. 184, no. 12, 2001, pages 1613-1616, ISSN: 0022-1899
- DATABASE BIOSIS [Online] BIOSCIENCES INFORMATION SERVICE, PHILADELPHIA, PA, US; 8 March 2001 (2001-03-08) VAJDY MICHAEL SEJR ET AL: "Potent mucosal HIV-1 specific immunity and protection from vaginal viral challenge following mucosal immunizations with non-replicating Sindbis virus-based particles." Database accession no. PREV200100258491 XP002212658 & FASEB JOURNAL, vol. 15, no. 5, 8 March 2001 (2001-03-08), page A1192 Annual Meeting of the Federation of American Societies for Experimental Biology on Experimental Biology 2001;Orlando, Florida, USA; March 31-April 04, 2001 ISSN: 0892-6638
- CAIRNS JS AND SARVER N: "New viral vectors for HIV vaccine delivery" AIDS RESEARCH AND HUMAN RETROVIRUSES, MARY ANN LIEBERT, US, vol. 14, no. 17, 20 November 1998 (1998-11-20), pages 1501-1508, XP002133046 ISSN: 0889-2229
- VACCINE, vol. 17, 1999, pages 2003-2008,
- J. VIROLOGY, vol. 73, 1999, pages 3723-3732,
- VACCINE, vol. 13, 1995, pages 1589-1595,
- VIROLOGY, vol. 212, 1995, pages 102-110,
- IMMUNOLOGY LETTERS, vol. 69, 1999, page 174,
- DATABASE BIOSIS [Online] BIOSCIENCES INFORMATION SERVICE, PHILADELPHIA, PA, US; Database accession no. PREV200000454057
- J. VIROLOGY, vol. 74, 2000, pages 11849-11857,
- J.VIROLOGY, vol. 70, 1996, pages 7910-7920,
- J.VIROLOGY, vol. 71, 1997, pages 248-258,
- J.VIROLOGY, vol. 70, 1996, pages 7900-7909,
- J.VIROLOGY, vol. 71, 1997, pages 2819-2829,
- PNAS, USA, vol. 96, 1999, pages 4598-4603,

## Description

### Technical Field

The present invention relates generally to mucosal immunization, for example, mucosal immunization using gene delivery systems. In particular to mucosal delivery of replication-defective vectors and/or recombinant alphavirus vectors and particles. Further, use of these systems for inducing potent mucosal, local and systemic immune responses following various routes of mucosal immunization is also described.

### Background of the Invention

Development of vaccines which invoke mucosal immunity against various pathogens would be desirable. Many disease-causing pathogens are transmitted through mucosal surfaces. For example, acquired immune deficiency syndrome (AIDS) is recognized as one of the greatest health threats facing modem medicine and worldwide sexual transmission of HIV is the leading cause of AIDS. There are, as yet, no cures or vaccines for AIDS.

In 1983-1984, three groups independently identified the suspected etiological agent of AIDS. See, e.g., Barre-Sinoussi et al. (1983) Science 220:868-871; Montagnier et al., in Human T-Cell Leukemia Viruses (Gallo, Essex & Gross, eds., 1984); Vilmer et al. (1984) The Lancet 1:753; Popovic et al. (1984) Science 224:497-500; Levy et al. (1984) Science 225:840-842. These isolates were variously called lymphadenopathy-associated virus (LAV), human T-cell lymphotropic virus type III (HTLV-III), or AIDS-associated retrovirus (ARV). All of these isolates are strains of the same virus, and were later collectively named Human Immunodeficiency Virus (HIV). With the isolation of a related AIDS-causing virus, the strains originally called HIV are now termed HIV-1 and the related virus is called HIV-2 See, e.g., Guyader et al. (1987) Nature 326:662-669; Brun-Vezinet et al. (1986) Science 233:343-346; Clavel et al. (1986) Nature 324:691-695. Consequently, there is a need in the art for compositions and methods suitable for treating and/or preventing HIV infection worldwide.

A great deal of information has been gathered about the HIV virus, and several targets for vaccine development have been examined including the *env, Gag, pol* and *tat* gene products encoded by HIV. Immunization with native and synthetic HIV-encoding polynucleotides has also been described, as described for example, in co-owned PCT/US99/31245 and references cited therein. In addition, polynucleotides encoding HIV have been administered in various attempts to identify a vaccine. (*See, e.g.,* Bagarazzi et al. (1999) J. Inject. Dis. 180:1351-1355; Wang et al. (1997) Vaccine 15:821-825). A replication-competent Venezuelan equine encephalitis (VEE) alphavirus vector carrying the matrix/capsid domain of HIV could elicit CTL responses has been administered subcutaneously in animals (Caley et al. (1997) J. Virol. 71:3031-3038). In addition, alphavirus vectors derived from Sindbis virus has also been shown to elicit HIV gag-specific responses in animals (Gardner et al. (2000) J. Virol. 74:11849-11857). Similarly, HIV peptides have also been administered to animal subjects. (Staats et al. (1997) AIDS Res Hum Retroviruses 13:945-952; Belyakov (1998) J. Clin.Invest. 102: 2072).

Recombinant alphavirus vectors and layered eukaryotic vector systems based on alphaviruses have also been described. (See, e.g., U.S. Patent Nos. 6,015,686; 6,015,694; 5,843,723). Hariharan et al. (1998) J. Virol. 72:950-958 reported that a single intramuscular immunization with pSIN vectors expressing the glycoprotein B of herpes simplex virus (HSV) type 1 induced a broad spectrum of immune responses, including virus-specific antibodies, cytotoxic T cells, and protection from lethal virus challenge in two different murine models. Polo et al. (1999) Proc. Natl. Acad. Sci. USA 96:4598-4603 reported similar protection in HSV challenge models following immunization with SIN replicon vectors particles rather than pSIN plasmid vectors. Tsuji et al. (1998) J. Viro/. 72:690-697 reported that subcutaneous administration in mice of recombinant SIN expressing a class I major histocompatibility complex-restricted 9-mer epitope of the *Plasmodium yoelii* circumsporozoite protein or the nucleoprotein of influenza virus induces a large epitope-specific CD8(+) T-cell response and provides a high degree of protection against infection with malaria or influenza A virus.

The prior art also includes Immunology Letters, vol. 69, 1999, page 174. This discloses influenza virus antigen gene (HA) containing VEE replicons packaged in VEE VLPs, which upon intranasal delivery led to protection against intranasal challenge with virulent influenza virus.

However, despite these and other studies, the utility of replication-defective gene delivery vehicles for mucosal immunization strategies that can protect against mucosal challenge has not been sufficiently defined. Thus, there remains a need for compositions and methods directed to treatment and prevention of various sexually transmitted pathogens.

### Summary of the Invention

The invention provides a replication-defective alphavirus replicon particle containing an alphavirus vector construct comprising a heterologous polynucleotide encoding at least one first antigen or modified form thereof, said first antigen or modified form thereof being capable of stimulating an immune response in a subject when administered intranasally, wherein said replication-defective alphavirus replicon particle is a chimeric alphavirus particle containing a Venezuelan Equine Encephalitis (VEE) virus vector construct packaged with Sindbis (SIN) virus envelope glycoproteins, for use in a therapeutic method of treating a mammalian subject by generating an immune response in the subject by intranasal administration.

The invention also provides use of a replication-defective alphavirus replicon particle containing an alphavirus vector construct comprising a heterologous polynucleotide encoding at least one first antigen or modified form thereof, said first antigen or modified form thereof being capable of stimulating an immune response in the subject when intranasally administered, wherein said replication-defective alphavirus replicon particle is a chimeric alphavirus particle containing a Venezuelan Equine Encephalitis (VEE) virus vector construct packaged with Sindbis (SIN) virus envelope glycoproteins, in the manufacture of a medicament for therapeutically treating a mammalian subject by generating an immune response in the subject by intranasal administration.

### Summary of the disclosure

Disclosed herein are gene delivery systems (*e.g*., recombinant alphavirus vectors) which are suitable for use in a variety of applications, including for example, mucosal immunization. Briefly stated, the disclosure relates to vector constructs and particles expressing antigens associated with one or more sexually transmitted disease pathogens, as well as methods of making and utilizing the same, particularly in protective mucosal immunization regimes. Preferably, the vectors are replication-defective, for example alphavirus vectors such as those derived from Sindbis. It is demonstrated herein thatantigen-specific protection against post-immunization challenge can be induced following mucosal administration of gene delivery vehicles (*e.g.*, alphavirus vectors) expressing the antigen.

In one aspect, the disclosure relates to a method of generating an immune response against an antigen. In certain embodiments, the method comprises mucosally administering to target cells of a subject, a replication-defective gene delivery vehicle (or vector) comprising a polynucleotide encoding at least one antigen (or modified form thereof), wherein the antigen (or modified form thereof) is capable of stimulating an immune response in the subject In certain embodiments, the target cells are in mucosal, local and/or systemic tissues. Mucosal administration can be, for example, intranasal, oral, intrarectal, and/or intravaginal administration. Preferably, for sexually transmitted pathogens, mucosal administration is by the intrarectal or intravaginal route. In certain embodiments, at least one antigen is derived, for example, from a sexually transmitted pathogen such as bacterial pathogen (*e.g.*, gonorrhea, chlamydia and syphilis) or a viral pathogen (*e.g*., HIV, HBV, HSV, HCV and HPV). In certain embodiments, the antigen(s) elicit(s) an HLA class I-restricted immune response and, optionally, also elicits an HLA Class II-restricted immune response.

In other aspects, the methods include delivery of genes encoding immune-enhancing cytokines, lymphokines, chemokines and the like. These genes can be inserted into the same gene delivery vehicle carrying the antigen(s) of interest (*e.g*., alphavirus replicon particle) or can be carried on one or more different gene delivery vehicles. In certain embodiments, the antigen(s) elicit(s) an HLA class I-restricted immune response and, optionally, also elicits an HLA Class II-restricted immune response.

The gene delivery vehicle (or vector) can be, for example, a nonviral vector, a particulate carrier (*e.g*., gold or tungsten particles coated with the polynucleotide and delivered using a gene gun); a liposome preparation; viral vector; a retroviral vector, or an alphavirus-derived vector. In certain aspects, an alphavirus-derived vector, for example a vector derived from Sindbis virus, Semliki Forest virus, Venezuelan Equine Encephalitis virus, Ross River virus or vector chimeras derived from any number of different alphaviruses (*e.g.*, SIN-VEE chimeras) are used. Any of the gene delivery vectors described herein (*e.g*., alphavirus vector) can be delivered, for example, to antigen presenting cells (APCs) such as dendritic cells. In certain embodiments, the subject and/or the cells is a mammal, for example a human. In any of the methods described herein, the target cells can be infected *in vivo*. Further, in any of the methods described herein, prior or subsequent to the step of administering to target cells, a nucleic acid molecule which encodes either Class I or Class II MHC protein, or combinations thereof, or a protein selected from the group consisting of CD3, ICAM-1, LFA-3 or analogues thereof can also be administered to the target cells. Furthermore, the above-described gene delivery vehicle for mucosal vaccination may be used in combination with one or more additional immunogenic compositions (gene delivery vehicle, polypeptide, protein, chemokine, cytokine, etc.) in which the one or more additional compositions are delivered by a mucosal or non-mucosal route(s).

These and other aspects of the present disclosure will become evident upon reference to the following detailed description and attached drawings. In addition, various references are set forth below which describe in more detail certain procedures or compositions (*e.g*., plasmids, etc.).

### Brief Description of the Drawings

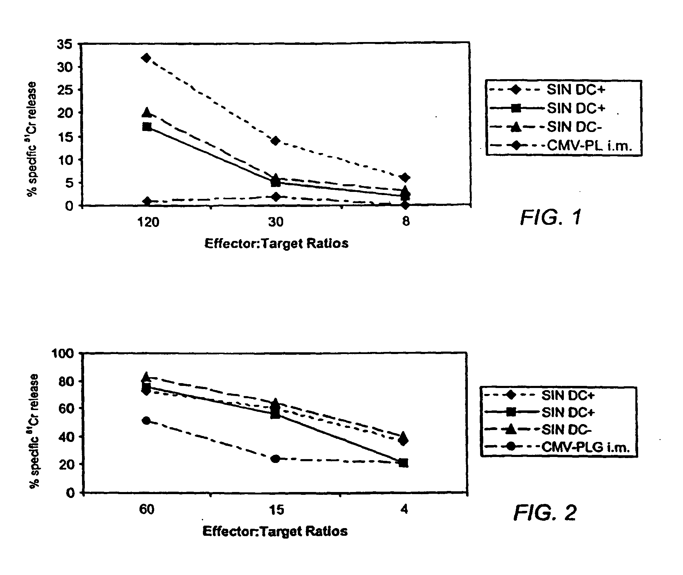

Figure 1 is a graph depicting local HIV-1 gag-specific CTL responses in cervical lymph nodes following intranasal immunization with gag-expressing SIN replicon particles as measured in a ⁵¹Cr release assay. "--◆--" depicts a first group of SIN DC+ animals; "—■—" depicts a second group of SIN DC+ animals; "--□--" depicts SIN DC-; and "—◆—" depicts a control plasmid (with CMV promoter) delivered intramuscularly.
Figure 2 is a graph depicting systemic gag-specific CTL responses in spleen following intranasal administration with SIN replicon particles as measured in a ⁵¹Cr release assay. "--◆--" depicts a first group of SIN DC+ animals; "-■-" depicts a second group of SIN DC+ animals; "--□--" depicts SIN DC- animals; and "— -●-—" depicts a control plasmid (with CMV promoter) delivered intramuscularly.

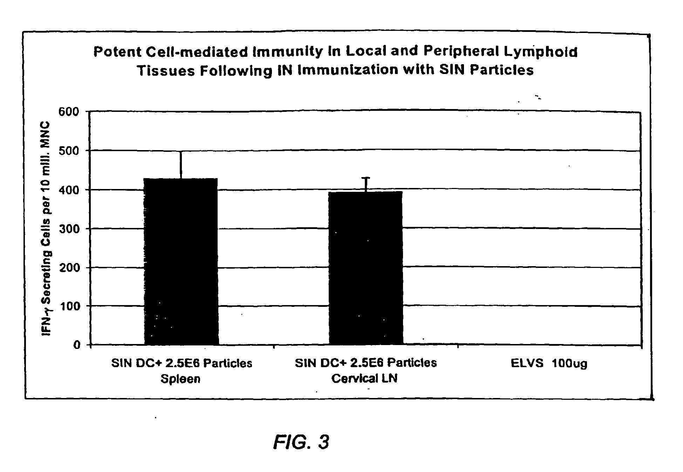

Figure 3 is a graph depicting number of INF-γ secreting cells in local and peripheral lymphoid tissue following intranasal immunization with SIN particles.

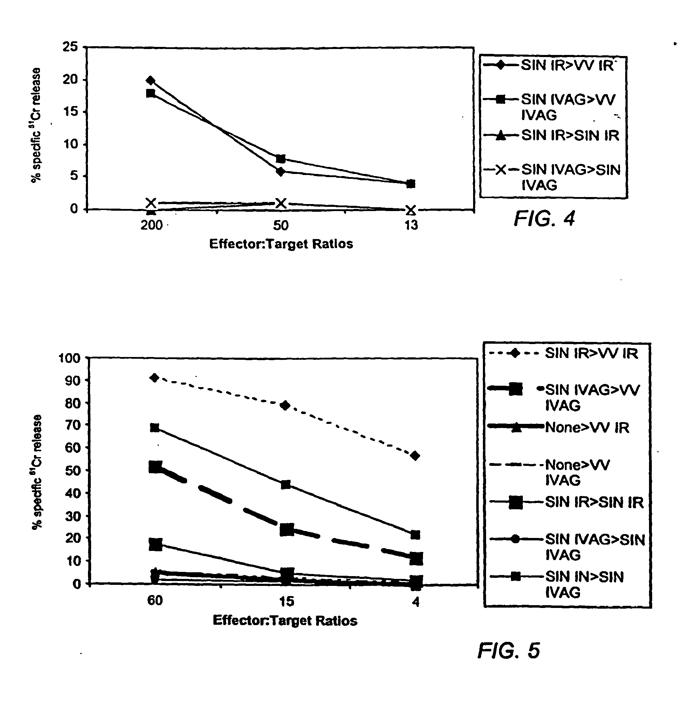

Figure 4 is a graph depicting local HIV-1 gag-specific CTL responses in iliac lymph nodes draining the rectal and vaginal mucosa following intrarectal (IR) or intra-vaginal (IVAG) administration of SIN replicon particles, as measured in a "Cr release assay. "--◆--" depicts responses in a first group after intrarectal administration of SIN followed by intrarectal (IR) challenge with vaccinia virus (VV); "—■—" depicts intravaginal administration of SIN followed by intravaginal challenge with VV; "—▲—" depicts responses in a second group following IR administration of SIN followed by challenge with VV; and "—X—" depicts a control plasmid (with CMV promoter) delivered intramuscularly.
Figure 5 is a graph depicting systemic HIV-1 gag-specific CTL responses in spleen following intra-rectal (IR) or intra-vaginal (IVAG) administration of SIN replicon particles, as measured in a "Cr release assay. "--◆--" depicts responses in a first group of animals after intrarectal administration of SIN replicons followed by intrarectal challenge with vaccinia virus (VV); "-■-" depicts intravaginal administration of SIN followed by intravaginal challenge with VV; "—■—" depicts animals challenged intravaginally with VV with no immunization; "—■—" depicts responses in animals after intrarectal administration of SIN replicons followed by intrarectal administration of SIN replicons; "—●—" depicts responses in animals after intravaginal administration of SIN replicons followed by intravaginal administration of SIN replicons; "—■—" depicts responses in animals after intranasal administration of SIN replicons followed by intravaginal administration of SIN replicons;

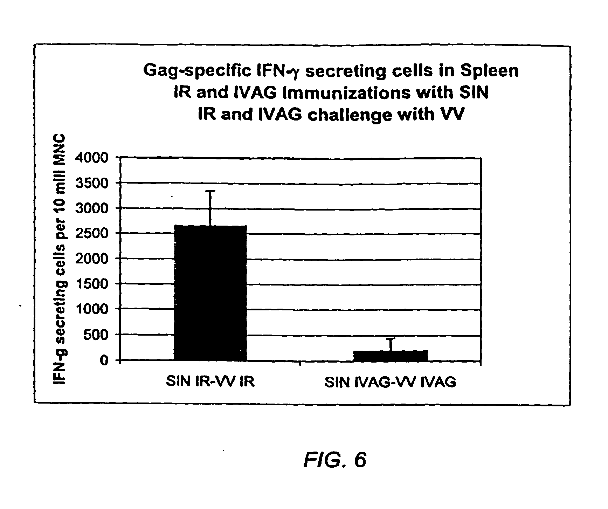

Figure 6 is a graph depicting IFN-γ secreting cells in spleen tissue following intrarectal (IR) and intravaginal (IVAG) immunization with SIN particles and IR and IVAG challenge with vaccinia virus (VV).

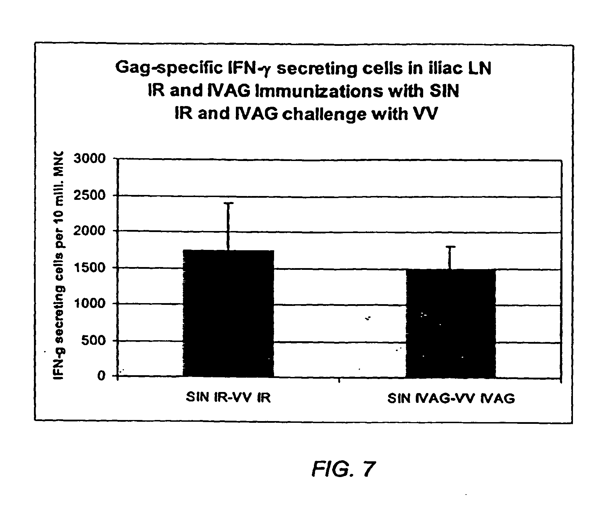

Figure 7 is a graph depicting IFN-γ secreting cells in iliac lymph nodes following intrarectal (IR) and intravaginal (IVAG) immunization with SIN particles and IR and IVAG challenge with vaccinia virus (VV).

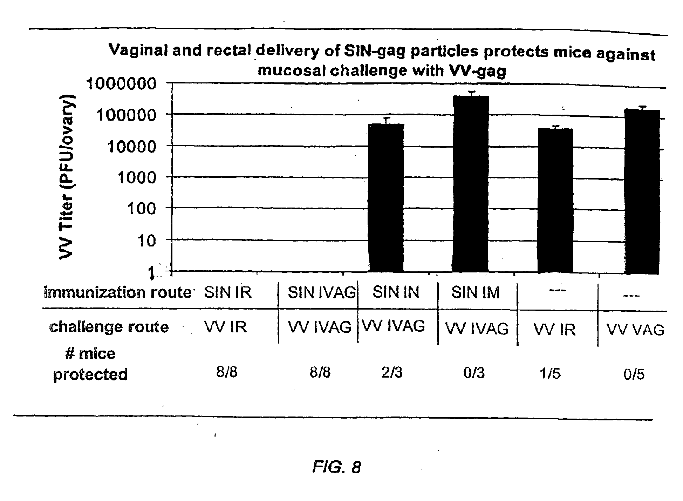

Figure 8 is a graph depicting vaccinia virus (VV) titer following vaginal and rectal delivery of SIN-gag particles.

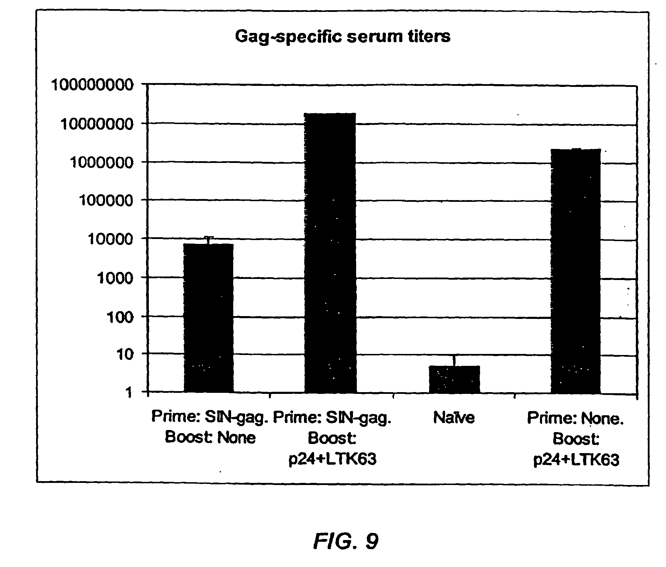

Figure 9 is a graph depicting gag-specific serum titers. From left to right, the bars show titers in animals: primed with SIN-gag and no boost; primed with SIN-gag and boosted with p24 polypeptide and LTK63 adjuvant; naïve animals; and no prime but subsequent boost with p24 and LTK63 adjuvant.

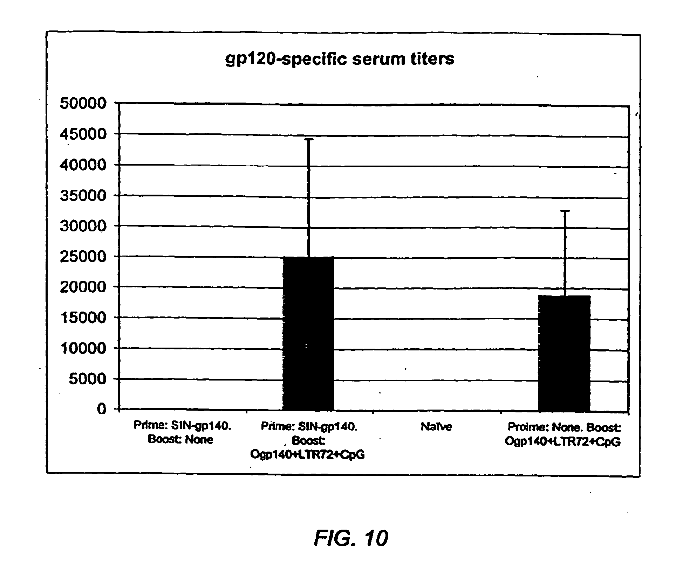

Figure 10 is a graph depicting gp120-specific serum titers. From left to right, the bars show titers in animals: primed with SIN-gp140 and no boost; primed with SIN-gag and boosted with gp-140 polypeptide and LTK72 adjuvant and CpG; naïve animals; and no prime but subsequent boost with gp-140 polypeptide and LTK72 adjuvant and CpG.

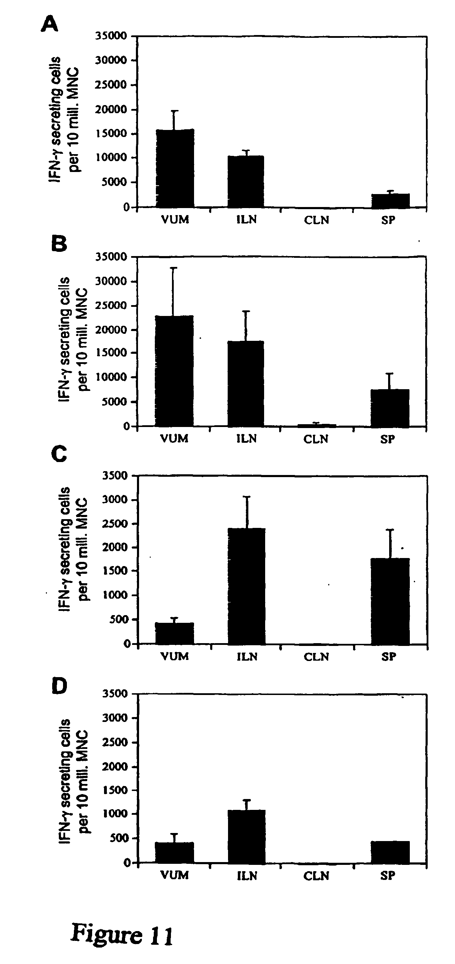

Figure 11, panels A to D, are graphs depicting Induction of local and systemic cellular immune responses after IN (A), IM (B), IR (C) and IVAG (D) immunizations with SIN-gag particles followed by IVAG challenge with VV-gag. IN and IM immunizations induced several fold higher numbers of gag-specific IFN-γ secreting cells in VUM and ILN compared to IR and IVAG immunizations. The mice were immunized 3 times IN or IM with 2.5x10⁶ SIN-gag particles and IR or IVAG with 10⁷ SIN-gag particles, three weeks later they were challenged vaginally with 10⁷ pfu of VV-gag, and sacrificed 5 days later. The results are shown as average number of p7g-specific IFN-γ secreting cells per 10 million mononuclear cells (MNC) of three independent experiments ± SD.

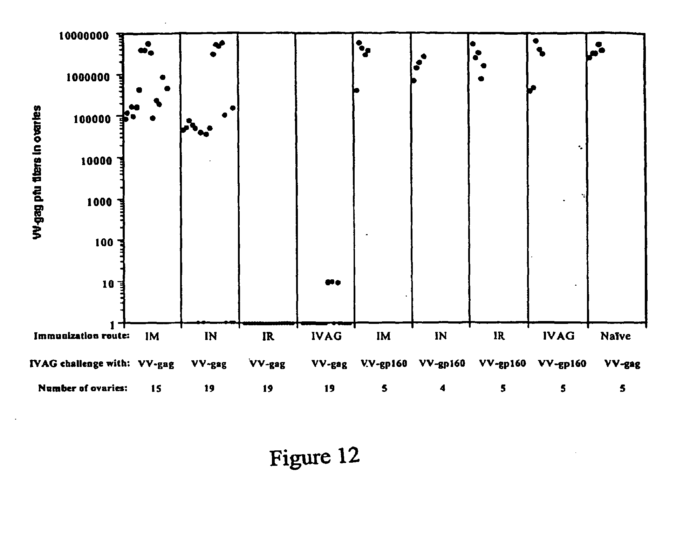

Figure 12 depicts Protection in ovaries from vaginal challenge with VV-gag following vaginal or rectal immunizations with SIN-gag particles. The mice were immunized 3 times IN or IM with 2.5x10⁶ SIN-gag particles and IR or IVAG with 10⁷ SIN-gag particles, three weeks later they were challenged vaginally with 10⁷ pfu of VV-gag, and sacrificed 5 days later. Ovaries were collected and standard pfu assay for determination of VV titers was performed. Each dot represents the numbers of plaque forming units per ovary/each mouse. As control naïve mice were challenged with VV-gag and mice immunized with SIN-gag particles were challenged with VV-gp160 and in both cases high pfu titers were evident in the ovaries.

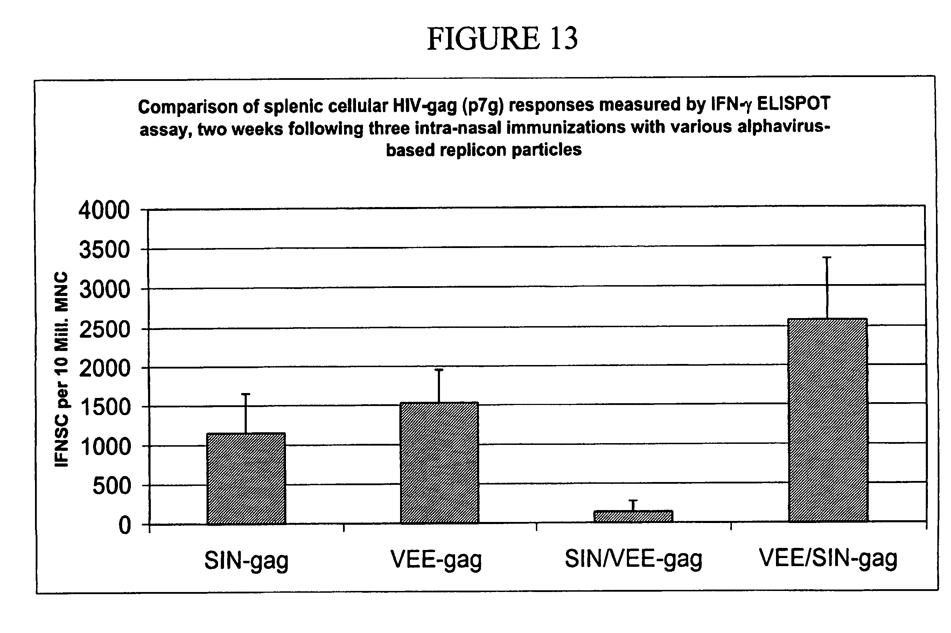

Figure 13 is a graph depicting the induction of an immunological response (as measured by IFN-γ ELISPOT assay) following intranasal immunization with alphavirus vector particles. Actual numbers represented by the bar graph are as follows: SIN-gag replicon particles gave 1154 (± 499); VEE-gag replicon particles 1530 (± 425); SIN/VEE-gag 140 (± 140); and VEE/SIN-gag 2586 (± 762).

### Detailed Description

The practice of the present invention will employ, unless otherwise indicated, conventional methods of chemistry, biochemistry, molecular biology, immunology and pharmacology, within the skin of the art. Such techniques are explained fully in the literature. See, e.g., Remington's Pharmaceutical Sciences, 18th Edition (Easton, Pennsylvania: Mack Publishing Company, 1990); Methods In Enzymology (S. Colowick and N. Kaplan, eds., Academic Press, Inc.); and Handbook of Experimental Immunology, Vols. I-IV (D.M. Weir and C.C. Blackwell, eds.,1986, Blackwell Scientific Publications); Sambrook, et al., Molecular Cloning: A Laboratory Manual (2nd Edition, 1989); Short Protocols in Molecular Biology, 4th ed. (Ausubel et al. eds., 1999, John Wiley & Sons); Molecular Biology Techniques: An Intensive Laboratory Course, (Ream et al., eds., 1998, Academic Press); PCR (Introduction to Biotechniques Series), 2nd ed. (Newton & Graham eds., 1997, Springer Verlag); Peters and Dalrymple, Fields Virology (2d ed), Fields et al. (eds.), B.N. Raven Press, New York, NY.

As used in this specification and the appended claims, the singular forms "a," "an" and "the" include plural references unless the content clearly dictates otherwise. Thus, for example, reference to "an antigen" includes a mixture of two or more such agents.

Prior to setting forth the invention, it may be helpful to an understanding thereof to first set forth definitions of certain terms that will be used hereinafter.

"Gene transfer" or "gene delivery" refers to methods or systems for reliably inserting DNA of interest into a host cell. Such methods can result in transient expression of non-integrated transferred DNA, extrachromosomal replication and expression of transferred replicons (e.g., episomes), or integration of transferred genetic material into the genomic DNA of host cells. Gene delivery expression vectors include, but are not limited to, vectors derived from alphaviruses, pox viruses and vaccinia viruses. When used for immunization, such gene delivery expression vectors may be referred to as vaccines or vaccine vectors.

The terms "Replication-defective" and "replication-incompetent" are used interchangeably to refer to a gene-delivery vehicle such as a viral vector, that does not make or propogate additional infectious, viral particles after being administered to a target cell. As will be apparent to those of skill in the art, the polynucleotides (*e.g.*, RNA) contained in the administered vector or particles may amplify or replicate, however, new progeny vector or viral particles are not formed and do not spread from cell to cell after administration.

"Alphavirus vector construct" refers to an assembly that is capable of directing the expression of a sequence(s) or gene(s) of interest. As described, for example, in U.S. Patent No. 6,015,695; U.S. Patent No. 6,015,686; U.S. Patent No. 5,842,723, and WO 97/38087, the vector construct should include a 5' sequence which is capable of initiating transcription of an alphavirus, as well as sequence(s) which, when expressed, code for biologically active alphavirus non-structural proteins (*e.g*., NSP1, NSP2, NSP3, and NSP4), and an alphavirus RNA polymerase recognition sequence. In addition, the vector construct should include a viral junction promoter region that may, in certain embodiments, be modified in order to prevent, increase, or reduce viral transcription of the subgenomic fragment. The vector may also include nucleic acid molecule(s) which are of a size sufficient to allow production of viable viral vector particles, a 5' promoter which is capable of initiating the synthesis of viral RNA *in vitro* or *in vivo* from cDNA, as well as one or more restriction sites, means for expressing multiple antigens (*e.g*., IRES element) and a polyadenylation sequence. Any of the sequences making up the alphavirus vector construct may be derived from one or more alphaviruses. As an RNA molecule, the alphavirus vector construct may also be referred to as an "RNA replicon."

"Structural protein expression cassette" refers to a recombinantly produced molecule that is capable of expressing alphavirus structural protein(s). The expression cassette must include a promoter and a sequence encoding alphavirus structural protein(s). Optionally, the expression cassette may include transcription termination, splice recognition, and polyadenylation addition sites. Preferred promoters include the CMV and adenovirus VA1RNA promoters, as well as alphavirus subgenomic junction region promoters. In addition, the expression cassette may contain selectable markers such as Neo, SV2 Neo, hygromycin, phleomycin, histidinol, and DHFR.

"Recombinant alphavirus particle" refers to a capsid that contains an alphavirus vector construct. Preferably, the alphavirus capsid is contained within a lipid bilayer, such as a cell membrane, in which viral encoded proteins (*e.g.,* envelope proteins) are embedded. A variety of vectors may be contained within the alphavirus particle, including the alphavirus vector constructs of the present disclosure. In addition, the recombinant alphavirus particle may be a chimera, containing elements from any number of different alphaviruses (*e.g*., RNA vector construct from SIN with capsid and/or envelope proteins from VEE). (See, also co-owned U.S. Patent No. 6,329,201).

The terms "polypeptide" and "protein" refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include postexpression modifications of the polypeptide, for example, glycosylation, acetylation, phosphorylation and the like. Furthermore, for purposes of the present invention, a "polypeptide" refers to a protein that includes modifications, such as deletions, additions and substitutions (generally conservative in nature), to the native sequence, so long as the protein maintains the desired activity. These modifications may be deliberate, as through site-directed mutagenesis, or may be accidental, such as through mutations of hosts that produce the proteins or errors due to PCR amplification.

An "antigen" refers to a molecule containing one or more epitopes (either linear, conformational or both) that will stimulate a host's immune system to make a humoral and/or cellular antigen-specific response. The term is used interchangeably with the term "immunogen." Normally, a B-cell epitope will include at least about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide that expresses an antigen or antigenic determinant *in vivo,* such as in gene therapy and DNA immunization applications, is also included in the definition of antigen herein.

For purposes of the present invention, antigens can be derived from any of several known viruses, bacteria, parasites and fungi, as described more fully below. The term also intends any of the various tumor antigens. Preferably, the antigens are derived from a sexually transmitted pathogen, for example a virus or a bacteria. Furthermore, for purposes of the present invention, an "antigen" refers to a protein that includes modifications, such as deletions, additions and substitutions (generally conservative in nature), to the native sequence, so long as the protein maintains the ability to elicit an immunological response, as defined herein. These modifications may be deliberate, as through site-directed mutagenesis, or may be accidental, such as through mutations of hosts that produce the antigens.

An "immunological response" to an antigen or composition is the development in a subject of a humoral and/or a cellular immune response to an antigen present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells. In addition, a chemokine response may be induced by various white blood or endothelial cells in response to an administered antigen.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art. See, e.g., Erickson et al., J. Immunol. (1993) 151:4189-4199; Doe et al., Eur. J. Immunol. (1994) 24:2369-2376. Recent methods of measuring cell-mediated immune response include measurement of intracellular cytokines or cytokine secretion by T-cell populations (*e.g*., by ELISPOT technique), or by measurement of epitope specific T-cells (e.g., by the tetramer technique)(reviewed by McMichael, A.J., and O'Callaghan, C.A., J. Exp. Med. 187(9):1367-1371, 1998; Mcheyzer-Williams, M.G., et al, Immunol. Rev. 150:5-21, 1996; Lalvani, A., et al, J. Exp. Med. 186:859-865, 1997).

Thus, an immunological response as used herein may be one that stimulates the production of CTLs, and/or the production or activation of helper T- cells. The production of chemokines and/or cytokines may also be stimulated. The antigen of interest may also elicit an antibody-mediated immune response. Hence, an immunological response may include one or more of the following effects: the production of antibodies by B-cells; and/or the activation of suppressor, cytotoxic, or helper T-cells and/or γδ T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

An "immunogenic composition" is a composition that comprises an antigenic molecule where administration of the composition to a subject results in the development in the subject of a humoral and/or a cellular immune response to the antigenic molecule of interest. The immunogenic composition can be introduced directly into a recipient subject, such as by injection, inhalation, oral, intranasal or any other mucosal (*e.g*., intra-rectally or intra-vaginally) route of administration.

By "subunit vaccine" is meant a vaccine composition that includes one or more selected antigens but not all antigens, derived from or homologous to, an antigen from a pathogen of interest such as from a virus, bacterium, parasite or fungus. Such a composition is substantially free of intact pathogen cells or pathogenic particles, or the lysate of such cells or particles. Thus, a "subunit vaccine" can be prepared from at least partially purified (preferably substantially purified) immunogenic polypeptides from the pathogen, or analogs thereof. The method of obtaining an antigen included in the subunit vaccine can thus include standard purification techniques, recombinant production, or synthetic production.

An "immuno-modulatory factor" refers to a molecule, for example a protein that is capable of modulating an immune response. Non-limiting examples of immunomodulatory factors include lymphokines (also known as cytokines), such as IL-6, TGF-β, IL-1, IL-2, IL-3, etc.); and chemokines (*e.g*., secreted proteins such as macrophage inhibiting factor). Certain cytokines, for example TRANCE, flt-3L, and a secreted form of CD40L are capable of enhancing the immunostimulatory capacity of APCs. Non-limiting examples of cytokines which may be used alone or in combination in the practice of the present invention include, interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-1α), interleukin-11 (IL-11), MIP-1γ, leukemia inlubitory factor (LIF), c-kit ligand, thrombopoietin (TPO), CD40 ligand (CD40L), tumor necrosis factor-related activation-induced cytokine (TRANCE) and flt3 ligand (flt-3L). Cytokines are commercially available from several vendors such as, for example, Genzyme (Framingham, MA), Amgen (Thousand Oaks, CA), R&D Systems and Immunex (Seattle, WA). The sequences of many of these molecules are also available, for example, from the GenBank database. It is intended, although not always explicitly stated, that molecules having similar biological activity as wild-type or purified cytokines (e.g., recombinantly produced or mutants thereof) and nucleic acid encoding these molecules are intended to be used.

By "subject" is meant any member of the subphylum chordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are intended to be covered. The system described above is intended for use in any of the above vertebrate species, since the immune systems of all of these vertebrates operate similarly.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

### A. GENE DELIVERY SYSTEMS

### 1. Alphavirus Vectors and Particles

As noted above, the present disclosure relates to alphavirus vector constructs, alphavirus particles containing such constructs, as well as methods for making and utilizing such vector constructs and particles in mucosal immunizations. The use of alphavirus based, replication-defective replicon particles, as a mucosal vaccine delivery system provides a number of advantages. Perhaps the most important aspect of immunization with a replication-defective virus vector is the control of gene product at the site of immunization. Immunization with replicating vectors, including the reported live, attenuated alphavirus Venezuelan equine encephalitis-based (VEE) vectors (Caley et al. (1997) J. Virol. 71:3031-3038; Davis et al. (1996) J. Virol. 70:3781-3787), results in the dissemination of the vector throughout the host. In contrast, immunization with a replication-defective vector results in the local expression, without the spread of newly propagated progeny vector. Moreover, the deficiency in replication reduces the risk of unwarranted inflammatory responses at sites of immunization (Villacres (2000) Virology, 270:54).

Alphavirus vectors suitable for use in the present disclosure can be constructed by any means, for example, as described in U.S. Patent Nos: 6,015,686; 6,015,694; 5,789,245 and 5,842,723. Briefly, sequences encoding alphavirus suitable for use in preparing the above-described vector constructs and particles may be readily obtained given the disclosure provided herein from naturally-occurring sources, or from depositories (*e.g*., the American Type Culture Collection, Rockville, Maryland). Representative examples of suitable alphaviruses include Aura (ATCC VR-368), Bebaru virus (ATCC VR-600, ATCC VR-1240), Cabassou (ATCC VR-922), Chikungunya virus (ATCC VR-64, ATCC VR-1241), Eastern equine encephalomyelitis virus (ATCC VR-65, ATCC VR-1242), Fort Morgan (ATCC VR-924), Getah virus (ATCC VR-369, ATCC VR-1243), Kyzylagach (ATCC VR-927), Mayaro (ATCC VR-66), Mayaro virus (ATCC VR-1277), Middleburg (ATCC VR-370), Mucambo virus (ATCC VR-580, ATCC VR-1244), Ndumu (ATCC VR-371), Pixuna virus (ATCC VR-372, ATCC VR-1245), Ross River virus (ATCC VR-373, ATCC VR-1246), Semliki Forest (ATCC VR-67, ATCC VR-1247), Sindbis virus (ATCC VR-68, ATCC VR-1248), Tonate (ATCC VR-925), Triniti (ATCC VR-469), Una (ATCC VR-374),

Venezuelan equine encephalomyelitis (ATCC VR-69), Venezuelan equine encephalomyelitis virus (ATCC VR-923, ATCC VR-1250 ATCC VR-1249, ATCC VR-532), Western equine encephalomyelitis (ATCC VR-70, ATCC VR-1251, ATCC VR-622, ATCC VR-1252), Whataroa (ATCC VR-926), and Y-62-33 (ATCC VR-375). Sequences obtained from one or more alphaviruses can be used in the same vector and/or particle. Within one preferred aspect of the present disclosure the sequences that encode wild-type alphavirus are obtained from a Sindbis virus.

The alphavirus vector component typically includes self-amplifying RNA (replicon) in which one or more of the structural protein genes of the virus are replaced by one or more genes of interest (*e.g*., antigens). RNA-based vectors, including alphavirus vectors, are also known as "replicons" because they retain the replicase functions necessary for RNA self-amplification and high-level expression, and can be launched *in vivo* following transfection with plasmid DNA (Dubensky et al. (1996) J. Virol. 70:508-519) or infection with virus-like particles. Alphavirus vector constructs will also typically contain inactivated, tandem or modified viral junction regions. Other modifications and methods of constructing suitable alphavirus vectors are described, for example, in U.S. Patent No. 6,015,686 and WO 97/38087.

The replicon RNA of the alphavirus vector construct may be delivered directly to the target cells by physical means or first packaged into particles. Generally, structural proteins necessary for packaging are supplied *in trans* by helper constructs or by packaging cell lines. The structural proteins may be homologous (*e.g*., derived from the same alphavirus as the vector construct); heterologous (*e.g*., derived from an alphavirus different from the vector construct); and hybrid (*e.g*., containing elements of multiple alphaviruses). Importantly, only the replicon RNA is packaged into the particle, as the helper RNAs typically lack the cis-acting packaging sequence required for encapsidation. Thus, alphavirus particles can be generated which infect target cells in culture or *in vivo*, and can express the gene of interest to high level; however, they are defective in that they lack critical portions of the alphavirus genome necessary to produce virus particles which could spread to other cells. In this way, when the replicon RNA is introduced into a suitable host cell, it self-amplifies, and the gene of interest is expressed from the subgenomic mRNA. However, no new virion particles are assembled *in vivo* due to the absence of structural protein genes.

Although no direct comparative studies among the various alphavirus replicons have been performed, differences in natural cell tropism are known to exist. For example, the lymphotropic VEE recently was shown to transduce murine DC (MacDonald et al. (2000) J. Virol. 74:914-922), while SIN and SFV are not lymphotropic. Infection of human DC has recently been demonstrated for alphaviruses or their derived vectors (Gardner et al. (2000) J. Virol. 74:11849-11857; WO 00/61772). Gardner identified SIN variants that are highly efficient for growth in immature human DC. The genetic determinant of human DC tropism for the SIN variant has been mapped to a single amino acid substitution in glycoprotein E2, and using this information, alphavirus replicon particles that can be used to target human DC can be generated. Detailed characterization of replicon infected DC both *in vitro* and *in vivo* revealed that the replicon-infected cells maintained their developmental and antigen presenting capabilities, indicating the potential utility of the DC-targeted SIN replicons for vaccine applications against infectious and malignant disease.

### 2. Additional Gene Delivery Vehicles

As noted above, mucosal genetic immunization has been shown to elicit both local and systemic immune responses. However, safety issues associated with revertants and potential infection hazards limits the utility of these systems. Moreover, replicating virus-based vector may spread rapidly throughout the host from the site of administration and cause unwarranted inflammatory responses at other sites. Therefore, the present disclosure describes viral and non-viral based genetic delivery systems that are replication-defective in the host and, accordingly, do not bear the possibility of reverting to an infectious state. Thus, the disclosure relates to compositions and methods of mucosal immunization using any replication-defective gene transfer vehicle.

A number of viral based systems have been developed for gene transfer into mammalian cells. For example, retroviruses provide a convenient platform for gene delivery systems. Selected sequences can be inserted into a vector and packaged in retroviral particles using techniques known in the art. The recombinant virus can then be isolated and delivered to cells of the subject either *in vivo* or *ex vivo.* A number of retroviral systems have been described (U.S. Patent No. 5,219,740; Miller and Rosman, BioTechniques (1989) 7:980-990; Miller, A.D., Human Gene Therapy (1990) 1:5-14; Scarpa et al., Virology (1991) 180:849-852; Burns et al., Proc. Natl. Acad Sci. USA (1993) 90:8033-8037; and Boris-Lawrie and Temin, Cur. Opin. Genet. Develop. (1993) 3:102-109.

A number of adenovirus vectors have also been described. Unlike retroviruses which integrate into the host genome, adenoviruses persist extrachromosomally thus minimizing the risks associated with insertional mutagenesis (Haj-Ahmad and Graham, J. Virol. (1986) 57:267-274; Bett et al., J. Virol. (1993) 67:5911-5921; Mittereder et al., Human Gene Therapy (1994) 5:717-729; Seth et al., J. Virol. (1994) 68:933-940; Barr et al., Gene Therapy (1994) 1:51-58; Berkner, K.L. BioTechniques (1988) 6:616-629; and Rich et al., Human Gene Therapy (1993) 4:461-476).

Additionally, various adeno-associated virus (AAV) vector systems have been developed for gene delivery. AAV vectors can be readily constructed using techniques well known in the art. See, e.g., U.S. Patent Nos. 5,173,414 and 5,139,941; International Publication Nos. WO 92/01070 (published 23 January 1992) and WO 93/03769 (published 4 March 1993); Lebkowski et al., Molec. Cell. Biol. (1988) 8:3988-3996; Vincent et al., Vaccines 90 (1990) (Cold Spring Harbor Laboratory Press); Carter, B.J. Current Opinion in Biotechnology (1992) 3:533-539; Muzyczka, N. Current Topics in Microbiol. and Immunol. (1992) 158:97-129; Kotin, R.M. Human Gene Therapy (1994) 5:793-801; Shelling and Smith, Gene Therapy (1994) 1:165-169; and Zhou et al., J. Exp. Med. (1994) 179:1867-1875.

Another vector system useful for delivering polynucleotides, mucosally and otherwise, is the enterically administered recombinant poxvirus vaccines described by Small, Jr., P.A., et al. (U.S. Patent No. 5,676,950, issued October 14, 1997) as well as the vaccinia virus and avian poxviruses. By way of example, vaccinia virus recombinants expressing the genes can be constructed as follows. The DNA encoding the particular synthetic Gag/antigen coding sequence is first inserted into an appropriate vector so that it is adjacent to a vaccinia promoter and flanking vaccinia DNA sequences, such as the sequence encoding thymidine kinase (TK). This vector is then used to transfect cells that are simultaneously infected with vaccinia. Homologous recombination serves to insert the vaccinia promoter plus the gene encoding the coding sequences of interest into the viral genome. The resulting TK recombinant can be selected by culturing the cells in the presence of 5-bromodeoxyuridine and picking viral plaques resistant thereto.

Alternatively, avipoxviruses, such as the fowlpox and canarypox viruses, can also be used to deliver the genes. Recombinant avipox viruses, expressing immunogens from mammalian pathogens, are known to confer protective immunity when administered to non-avian species. The use of an avipox vector is particularly desirable in human and other mammalian species since members of the avipox genus can only productively replicate in susceptible avian species and therefore are not infective in mammalian cells. Methods for producing recombinant avipoxviruses are known in the art and employ genetic recombination, as described above with respect to the production of vaccinia viruses. See, e.g., WO 91/12882; WO 89/03429; and WO 92/03545. Picorna virus-derived vectors can also be used. (See, e.g., U.S. Patent Nos. 5,614,413 and 6,063,384).

Molecular conjugate vectors, such as the adenovirus chimeric vectors described in Michael et al., J. Biol. Chem. (1993) 268:6866-6869 and Wagner et al., Proc. Natl. Acad. Sci. USA (1992) 89:6099-6103, can also be used for gene delivery.

A vaccinia based infection/transfection system can be conveniently used to provide for inducible, transient expression of the coding sequences of interest (for example, a synthetic Gag/HCV-core expression cassette) in a host cell. In this system, cells are first infected *in vitro* with a vaccinia virus recombinant that encodes the bacteriophage T7 RNA polymerase. This polymerase displays exquisite specificity in that it only transcribes templates bearing T7 promoters. Following infection, cells are transfected with the polynucleotide of interest, driven by a T7 promoter. The polymerase expressed in the cytoplasm from the vaccinia virus recombinant transcribes the transfected DNA into RNA that is then translated into protein by the host translational machinery. The method provides for high level, transient, cytoplasmic production of large quantities of RNA and its translation products. See, e.g., Elroy-Stein and Moss, Proc. Natl. Acad. Sci. USA (1990) 87:6743-6747; Fuerst et al., Proc. Natl. Acad. Sci. USA (1986) 83:8122-8126.

### 3. Amplification Systems

As an alternative approach to infection with vaccinia or avipox virus recombinants, or to the delivery of genes using other viral vectors, an amplification system can be used that will lead to high level expression following introduction into host cells. Specifically, a T7 RNA polymerase promoter preceding the coding region for T7 RNA polymerase can be engineered. Translation of RNA derived from this template will generate T7 RNA polymerase that in turn will transcribe more template. Concomitantly, there will be a cDNA whose expression is under the control of the T7 promoter. Thus, some of the T7 RNA polymerase generated from translation of the amplification template RNA will lead to transcription of the desired gene. Because some T7 RNA polymerase is required to initiate the amplification, T7 RNA polymerase can be introduced into cells along with the template(s) to prime the transcription reaction. The polymerase can be introduced as a protein or on a plasmid encoding the RNA polymerase. For a further discussion of T7 systems and their use for transforming cells, see, e.g., International Publication No. WO 94126911; Studier and Moffatt, J. Mol. Biol. (1986) 189:113-130; Deng and Wolff, Gene (1994) 143:245-249; Gao et al., Biochem. Biophys. Res. Commun. (1994) 200:1201-1206; Gao and Huang, Nuc. Acids Res. (1993) 21:2867-2872; Chen et al., Nuc. Acids Res. (1994) 22:2114-2120; and U.S. Patent No. 5,135,855.

### 4. Liposomal/Lipid Delivery Vehicles

The polynucleotide of interest can also be delivered without a viral vector. For example, packaged as DNA or RNA in liposomes prior to delivery to the subject or to cells derived therefrom. Lipid encapsulation is generally accomplished using liposomes that are able to stably bind or entrap and retain nucleic acid. The ratio of condensed DNA to lipid preparation can vary but will generally be around 1:1 (mg DNA:micromoles lipid), or more of lipid. For a review of the use of liposomes as carriers for delivery of nucleic acids, see, Hug and Sleight, Biochim. Biophys. Acta. (1991) 1097:1-17; Straubinger et al., in Methods of Enzymology (1983), Vol. 101, pp. 512-527.

Liposomal preparations for use according to the present disclosure include cationic (positively charged), anionic (negatively charged) and neutral preparations, with cationic liposomes particularly preferred. Cationic liposomes have been shown to mediate intracellular delivery of plasmid DNA (Felgner et al., Proc. Natl. Acad. Sci. USA (1987) 84:7413-7416); mRNA (Malone et al., Proc. Natl. Acad. Sci. USA (1989) 86:6077-6081); and purified transcription factors (Debs et al., J. Biol. Chem. (1990) 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner et al., Proc. Natl. Acad. Sci. USA (1987) 84:7413-7416). Other commercially available lipids include (DDAB/DOPE) and DOTAP/DOPE (Boerhinger). Other cationic liposomes can be prepared from readily available materials using techniques well known in the art. See, e.g., Szoka et al., Proc. Natl. Acad. Sci. USA (1978) 75:4194-4198; PCT Publication No. WO 90/11092 for a description of the synthesis of DOTAP (1,2-bis(oleoyloxy)-3-(trimethylammonio)propane) liposomes. Cationic microparticles can be prepared from readily available materials using techniques known in the art. See, *e.g.,* co-owned WO 01/136599.

Similarly, anionic and neutral liposomes are readily available, such as, from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See, e.g., Straubinger et al., in METHODS OF IMMUNOLOGY (1983), Vol. 101, pp. 512-527; Szoka et al., Proc. Natl. Acad. Sci. USA (1978) 75:4194-4198; Papahadjopoulos et al., Biochim. Biophys. Acta (1975) 394:483; Wilson et al., Cell (1979) 17:77); Deamer and Bangham, Biochim. Biophys. Acta (1976) 443:629; Ostro et al., Biochem. Biophys. Res. Commun. (1977) 76:836; Fraley et al., Proc. Natl. Acad. Sci. USA (1979) 76:3348); Enoch and Strittmatter, Proc. Natl. Acad. Sci. USA (1979) 76:145); Fraley et al., J. Biol. Chem. (1980) 255:10431; Szoka and Papahadjopoulos, Proc. Natl. Acad. Sci. USA (1978) 75:145; and Schaefer-Ridder et al., Science (1982) 215:166.

The DNA and/or protein antigen(s) can also be delivered in cochleate lipid compositions similar to those described by Papahadjopoulos et al., Biochem. Biophys. Acta. (1975) 394:483-491. See, also, U.S. Patent Nos. 4,663,161 and 4,871,488.

### 5. Particulate Carriers

The compositions may also be encapsulated, adsorbed to, or associated with, particulate carriers. Such carriers present multiple copies of a selected antigen to the immune system and promote migration, trapping and retention of antigens in local lymph nodes. The particles can be taken up by profession antigen presenting cells such as macrophages and dendritic cells, and/or can enhance antigen presentation through other mechanisms such as stimulation of cytokine release. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee JP, et al., J Microencapsul. 14(2):197-210,1997; O'Hagan DT, et al., Vaccine 11(2):149-54, 1993.

Furthermore, other particulate systems and polymers can be used for the *in vivo* or *ex vivo* delivery of the gene of interest. For example, polymers such as polylysine; polyarginine, polyomithine, spermine, spermidine, as well as conjugates of these molecules, are useful for transferring a nucleic acid of interest. Similarly, DEAE dextran-mediated transfection, calcium phosphate precipitation or precipitation using other insoluble inorganic salts, such as strontium phosphate, aluminum silicates including bentonite and kaolin, chromic oxide, magnesium silicate, talc, and the like, will find use with the present methods. See, e.g., Felgner, P.L., Advanced Drug Delivery Reviews (1990) 5:163-187, for a review of delivery systems useful for gene transfer. Peptoids (Zuckerman, R.N., et al., U.S. Patent No. 5,831,005, issued November 3, 1998) may also be used for delivery of a construct disclosed herein.

Additionally, biolistic delivery systems employing particulate carriers such as gold and tungsten, are especially useful for delivering synthetic expression cassettes As disclosed herein. The particles are coated with the synthetic expression cassette(s) to be delivered and accelerated to high velocity, generally under a reduced atmosphere, using a gun powder discharge from a "gene gun." For a description of such techniques, and apparatuses useful therefore, see, e.g., U.S. Patent Nos. 4,945,050; 5,036,006; 5,100,792; 5,179,022; 5,371,015; and 5,478,744. Also, needle-less injection systems can be used (Davis, H.L., et al, Vaccine 12:1503-1509, 1994; Bioject, Inc., Portland, OR).

### B. ANTIGENS

Any of the gene delivery vehicles described herein can contain one or more heterologous sequences encoding one or more heterologous gene products, particularly polypeptide antigens. Virtually any heterologous gene product or polypeptide can be used. Antigens that are particularly useful in the practice of the present invention include polypeptide antigens derived from sexually transmitted pathogens or other viruses that infect or are transmitted through mucosal surfaces. Non-limiting representative examples of sexually transmitted pathogens and antigens derived therefrom include antigens derived from bacterial pathogens (*e.g*., chlamydia, gonorrhea and syphilis) and viral pathogens (*e.g*., Human Immmunodeficiency Virus ("HIV"), Hepatitis B and C Virus ("HBV" and "HCV", respectively), Human Papiloma Virus ("HPV"), Herpes Simplex Virus ("HSV"), and the like). Non-limiting examples of other viruses that may be transmitted via mucosal surfaces include rhinovirus, influenza, respiratory syncytial virus (RSV), parainfluenza virus (PIV), and the like. As utilized within the context of the present invention, "immunogenic portion" refers to a portion of the respective antigen that is capable, under the appropriate conditions, of causing an immune response (*i.e*., cell-mediated or humoral). "Portions" may be of variable size, but are preferably at least 9 amino acids long, and may include the entire antigen. Cell-mediated immune responses may be mediated through Major Histocompatability Complex ("MHC") class I presentation, MHC Class II presentation, or both.

The genes of HIV are located in the central region of the proviral DNA and encode at least nine proteins divided into three major classes: (1) the major structural proteins, Gag, Pol, and Env; (2) the regulatory proteins, Tat and Rev and (3) the accessory proteins, Vpu, Vpr, Vif, and Nef. Although exemplified herein with relation to antigens obtained from HIV_{SF2}, sequence obtained from other HIV variants may be manipulated in similar fashion following the teachings of the present specification. Such other variants include, but are not limited to, Gag protein encoding sequences obtained from the isolates HIV_{IIIb}, HIV_{SF2}, HIV-1_{SF162}, HIV-1_{SF170}, HIV_{LAV}, HIV_{LA1}, HIV_{MN}, HIV-1 _{CM235},, HIV-1_{US4}, other HIV-1 strains from diverse subtypes (e.g., subtypes, A through G, and O), HIV-2 strains and diverse subtypes (e.g., HIV-2_{UC1} and HIV-2_{UC2}), and simian immunodeficiency virus (SIV). (See, e.g., Virology, 3rd Edition (W.K. Joklik ed. 1988); Fundamental Virology, 2nd Edition (B.N. Fields and D.M. Knipe, eds. 1991); Virology, 3rd Edition (Fields, BN, DM Knipe, PM Howley, Editors, 1996, Lippincott-Raven, Philadelphia, PA; for a description of these and other related viruses).

As will be evident to one of ordinary skill in the art, various immunogenic portions of the above-described antigens may be combined in order to induce an immune response when administered as described herein. Further, the antigen-encoding gene delivery vehicles can also be used in combination with one or more additional gene delivery vehicles and/or polypeptides.

In addition, due to the large immunological variability that is found in different geographic regions for the open reading frame of HIV, particular combinations of antigens may be preferred for administration in particular geographic regions. Briefly, at least eight different subtypes of HIV have been identified and, of these, subtype B viruses are more prevalent in North America, Latin America and the Caribbean, Europe, Japan and Australia. Almost every subtype is present in sub-Saharan Africa, with subtypes A and D predominating in central and eastern Africa, and subtype C in southern Africa. Subtype C is also prevalent in India and it has been recently identified in southern Brazil. Subtype E was initially identified in Thailand, and is also present in the Central African Republic. Subtype F was initially described in Brazil and in Romania. The most recent subtypes described are G, found in Russia and Gabon, and subtype H, found in Zaire and in Cameroon. Group O viruses have been identified in Cameroon and also in Gabon. Thus, as will be evident to one of ordinary skill in the art, it is generally preferred to construct a vector for administration that is appropriate to the particular HIV subtype that is prevalent in the geographical region of administration. Subtypes of a particular region may be determined by two-dimensional double immunodiffusion or, by sequencing the HIV genome (or fragments thereof) isolated from individuals within that region.

As described above, also presented by HIV are various *Gag* and *Env* antigens. HIV-1 *Gag* proteins are involved in many stages of the life cycle of the virus including, assembly, virion maturation after particle release, and early post-entry steps in virus replication. The roles of HIV-1 *Gag* proteins are numerous and complex (Freed, E.O. (1998) Virology 251:1-15).

Env coding sequences for use in the present invention include, but are not limited to, polynucleotide sequences encoding the following HIV-encoded polypeptides: gp 160, gp140, and gp 120 (see, e.g., U.S. Patent No. 5,792,459 for a description of the IDV-1_{SP2} ("SF2") Env polypeptide). The envelope protein of HIV-1 is a glycoprotein of about 160 kD (gp160). During virus infection of the host cell, gp160 is cleaved by host cell proteases to form gp120 and the integral membrane protein, gp41. The gp41 portion is anchored in (and spans) the membrane bilayer of virion, while the gp120 segment protrudes into the surrounding environment As there is no covalent attachment between gp120 and gp41, free gp120 is released from the surface of virions and infected cells. Thus, gp160 includes the coding sequences for gp120 and gp41. The polypeptide gp41 is comprised of several domains including an oligomerization domain (OD) and a transmembrane spanning domain (TM). In the native envelope, the oligomerization domain is required for the non-covalent association of three gp41 polypeptides to form a trimeric structure: through non-covalent interactions with the gp41 trimer (and itself), the gp120 polypeptides are also organized in a trimeric structure. A cleavage site (or cleavage sites) exists approximately between the polypeptide sequences for gp 120 and the polypeptide sequences corresponding to gp41. This cleavage site(s) can be mutated to prevent cleavage at the site. The resulting gp140 polypeptide corresponds to a truncated form of gp160 where the transmembrane spanning domain of gp41 has been deleted. This gp140 polypeptide can exist in both monomeric and oligomeric (*i.e*. trimeric) forms by virtue of the presence of the oligomerization domain in the gp41 moiety. In the situation where the cleavage site has been mutated to prevent cleavage and the transmembrane portion of gp41 has been deleted the resulting polypeptide product can be designated "mutated" gp140. As will be apparent to those in the field, the cleavage site can be mutated in a variety of ways. (See, also, WO 00/39302).

As noted above, at least one immunogenic portion of an HIV antigen may be incorporated into a gene delivery vehicle and used for mucosal immunization. The immunogenic portion(s) incorporated into the vehicle (*e.g*., alphavirus vector construct) may be of varying length, although it is generally preferred that the portions be at least 9 amino acids long and may include the entire antigen. Immunogenicity of a particular sequence is often difficult to predict, although T cell epitopes may be predicted utilizing computer algorithms such as TSITES (MedImmune, Maryland), in order to scan coding regions for potential T-helper sites and CTL sites. From this analysis, peptides are synthesized and used as targets in an *in vitro* cytotoxic assay. Other assays, however, may also be utilized, including, for example, ELISA, which detects the presence of antibodies against the newly introduced vector, as well as assays which test for T helper cells, such as gamma-interferon assays, IL-2 production assays and proliferation assays.

Immunogenic portions may also be selected by other methods. For example, the HLA A2.1 transgenic mouse has been shown to be useful as a model for human T-cell recognition of viral antigens. Briefly, in the influenza and hepatitis B viral systems, the murine T cell receptor repertoire recognizes the same antigenic determinants recognized by human T cells. In both systems, the CTL response generated in the HLA A2.1 transgenic mouse is directed toward virtually the same epitope as those recognized by human CTLs of the HLA A2.1 haplotype (Vitiello et al. (1991) J. Exp. Med. 173:1007-1015; Vitiello et al. (1992) Abstract of molecular Biology of Hepatitis B Virus Symposia*).*

Additional immunogenic portions of the HIV may be obtained by truncating the coding sequence at various locations including, for example, to include one or more epitopes from the various domains of the HTV genome. As noted above, such domains include structural domains such as *Gag, Gag-polymerase, Gag-protease, reverse transcriptase (RT), integrase (IN)* and *Env.* The structural domains are often further subdivided into polypeptides, for example, p55, p24, p6 *(Gag);* p160, p10, p15, p31, p65 *(pol, prot, RT and IN);* and gp160, gp120 and gp41 *(Env).* Additional epitopes of HIV and other sexually transmitted diseases are known or can be readily determined using methods known in the art. Also included in the disclosure aremolecular variants of such polypeptides, for example as described in PCT/US99/31245; PCT/US99/31273 and PCT/US99/31272.

Other sexually or mucosally transmitted diseases, both viral and bacterial, can also be addressed using the compositions and methods described herein. For example, it appears that in the case of HSV, vaginal immunization with vector expressing HSV antigens (*e.g*., gB, gD) may provide protection against vaginal. Thus, one or more antigens derived from HSV can be used in as described herein to treat and prevent HSV infection.

### 1. Preparation of Vectors Carrying Heterologous Gene Products

In certain embodiments, sequences encoding one or more antigens are incorporated into a gene delivery vehicle such as viral vector or particle. As will be evident to one of ordinary skill in the art given the disclosure provided herein, the efficiency of packaging and hence, viral titer, of various viral vectors is to some degree dependent upon the size of the sequence to be packaged. Thus, in order to increase the efficiency of packaging and the production of viable vector particles (*e.g.*, alphavirus vector particles), additional non-coding sequences may be added to the vector construct.

In certain applications of the vectors or resulting particles described herein, the expression of more than one heterologous gene is desired. For example, in order to treat sexually transmitted disease such as HIV, multiple administrations of vectors or particles, or administration of vectors or particles expression more than one gene product may be required. In addition, immunogenicity may be further enhanced by encoding both an antigen and an immunomodulator (*e.g*., cytokine, lymphokine, chemokine, etc.). Therefore, within one embodiment of the invention viral vectors (*e.g.*, alphavirus vectors) may be constructed by placing appropriate signals, such as ribosome readthrough or internal ribosome entry sites (IRES) between cistrons. Alternatively, multiple subgenomic junction region promoters (*e.g*., derived from alphavirus) can be utilized. Further, a vector construct may express (either separately or as one construct) all or immunogenic portions of various mucosally transmitted (*e.g*., sexually transmitted) pathogens.

Within one aspect of the invention, vector constructs (*e.g.*, alphavirus vectors and particles) are provided which direct the expression of immunogenic portions of HIV antigens. The integrated form of HIV-1, also known as the provirus, is approximately 9.8 kilobases in length. (see, e.g., Muesing et al. (1985) Nature 313:450-48). Both ends of the provirus are flanked by a repeated sequence known as the long terminal repeats (LTRs).

Sequences that encode the above-described proteins (*e.g*. antigens) may be readily obtained from a variety of sources, including for example, depositories such as the American Type Culture Collection (ATCC, Rockville, MD), or from commercial sources such as British Bio-Technology Limited (Cowley, Oxford, England). Representative examples of molecularly cloned genomes that encode the hepatitis B virus may be obtained from sources such as the American Type Culture Collection (ATCC, Rockville, MD). For example, ATCC No. 45020 contains the total genomic DNA of hepatitis B (extracted from purified Dane particles) (*see* Figure 3 of Blum et al., TIG 5(5):154-158, 1989) in the *Bam* HI site of pBR322 (Moriarty et al., Proc. Natl. Acad. Sci. USA 78:2606-2610, 1981).

Alternatively, sequences encoding the polypeptide of interest can be generated by the polymerase chain reaction (PCR). Mullis et al. (1987) Methods Enzymol. 155:335-350; PCR Protocols, A Guide to Methods and Applications, Innis et al (eds) Harcourt Brace Jovanovich Publishers, NY (1994)). This technique uses DNA polymerase, usually a thermostable DNA polymerase, to replicate a desired region of DNA. The region of DNA to be replicated is identified by oligonucleotides of specified sequence complementary to opposite ends and opposite strands of the desired DNA to prime the replication reaction. Repeated successive cycles of replication result in amplification of the DNA fragment delimited by the primer pair used. A number of parameters influence the success of a reaction. Among them are annealing temperature and time, extension time, Mg²⁺ and ATP concentration, pH, and the relative concentration of primers, templates, and deoxyribonucleotides.

Once coding sequences for desired proteins have been prepared or isolated, such sequences can be cloned into any suitable vector or replicon. Numerous cloning vectors are known to those of skill in the art, and the selection of an appropriate cloning vector is a matter of choice. Ligations to other sequences are performed using standard procedures, known in the art.

### 2. Polypeptide Preparation

Similarly, the selected coding sequences can be cloned into any suitable expression vector for expression. The expressed product can optionally be purified prior to mucosal administration. Briefly, a polynucleotide encoding these proteins can be introduced into an expression vector that can be expressed in a suitable expression system. A variety of bacterial, yeast, mammalian, insect and plant expression systems are available in the art and any such expression system can be used. Optionally, a polynucleotide encoding these proteins can be translated in a cell-free translation system. Such methods are well known in the art. The proteins also can be constructed by solid phase protein synthesis. If desired, the polypeptides also can contain other amino acid sequences, such as amino acid linkers or signal sequences, as well as ligands useful in protein purification, such as glutathione-S-transferase and staphylococcal protein A. Alternatively, antigens of interest can be purchased from commercial sources.

### C. DELIVERY

The compositions (*e.g*., gene delivery vehicles and optional polypeptide antigens) described herein can be delivered using any suitable means (*e.g.*, intravenously, intramuscularly, intraperitoneally, subcutaneously, orally, rectally, intraocularly, intranasally), or by various physical methods such as lipofection (Felgner et al. (1989) Proc. Natl. Acad. Sci. USA 84:7413-7417), direct DNA injection (Acsadi et al. (1991) Nature 352:815-818); microprojectile bombardment (Williams et al. (1991) PNAS 88:2726-2730); liposomes of several types (*see, e.g.,* Wang et al. (1987) PNAS 84:7851-7855); CaPO₄ (Dubensky et al. (1984) PNAS 81:7529-7533); DNA ligand (Wu et al (1989) J. of Biol. Chem. 264:16985-16987); administration of polypeptides alone; administration of nucleic acids alone (WO 90/11092); or administration of DNA linked to killed adenovirus (Curiel et al. (1992), Hum. Gene Ther. 3:147-154); via polycation compounds such as polylysine, utilizing receptor specific ligands; as well as with psoralen inactivated viruses such as Sendai or Adenovirus. In addition, the eukaryotic layered vector initiation systems may either be administered directly (*i.e., in vivo*), or to cells that have been removed (*ex vivo*), and subsequently returned.

In a preferred embodiment, the gene delivery vehicles and optional polypeptide-containing compositions are administered mucosally. Methods of mucosal delivery are known in the art, for example as described in *Remington's, supra.* Delivery of the compositions rectally and vaginally is particularly preferred in the case of sexually transmitted pathogens, as this mode of administration provides access to the cells first exposed to the pathogens. Similarly, intranasal administration may be preferred in diseases, like rhinovirus, that infect through nasal mucosa. In some instances, intranasal administration may induce immunity in the vaginal mucosa and oral immunization may induce immunity in the rectal mucosa. Moreover, combinations of various routes of mucosal administration and/or various routes of systemic administration can be used in order to induce optimal immunity and protection (both at the site the pathogen enters as well as at systemic sites where a mucosal pathogen has spread to. Additionally, mucosal administration eliminates the need for syringes or other administration devices. Dosage treatment may be a single dose schedule or a multiple dose schedule.

In certain embodiments, the replication-defective vectors are administered via nucleic acid immunization or the like using standard gene delivery protocols. Methods for gene delivery are known in the art. See, e.g., U.S. Patent Nos. 5,399,346, 5,580,859, 5,589,466. Alphavirus compositions can be delivered either directly to the vertebrate subject or, alternatively, delivered *ex vivo,* to cells derived from the subject and the cells reimplanted in the subject. In preferred embodiments, the compositions are delivered *in vivo*. Delivery of replication-defective compositions *in vivo* can generally be accomplished using any means known in the art, for example, by injection using either a conventional syringe, needless devices such as Bioject® or a gene gun, such as the Accell® gene delivery system (PowderJect Technologies, Inc., Oxford, England). The constructs can be delivered (*e.g*., injected) either subcutaneously, epidermally, intradermally, intramuscularly, intravenous, mucosally (such as nasally, rectally and vaginally), intraperitoneally, orally or combinations thereof.

Within other aspects, methods are disclosed for administering the gene delivery systems described herein, including recombinant alphavirus vectors or particles. Briefly, the final mode of viral vector administration usually relies on the specific therapeutic application, the best mode of increasing vector potency, and the most convenient route of administration. Generally, this embodiment includes compositions which can be designed to be delivered by, for example, (1) direct injection into the blood stream; (2) direct injection into a specific tissue or tumor; (3) oral administration; (4) nasal inhalation; (5) direct application to mucosal tissues (*e.g*., intranasally, intrarectally and/or intravaginally); and/or (6) *ex vivo* administration of transduced autologous cells into the animal. Thus the therapeutic alphavirus vector can be administered in such a fashion such that the vector can (a) transduce a normal healthy cell and transform the cell into a producer of a therapeutic protein or agent which is secreted systemically or locally, (b) transform an abnormal or defective cell, transforming the cell into a normal functioning phenotype, (c) transform an abnormal cell so that it is destroyed, and/or (d) transduce cells to manipulate the immune response.

The compositions disclosed herein can be administered alone or can be administered with one or more additional gene delivery vehicles and/or one or more proteins. In such embodiments, the multiple compositions can be administered in any order, for example gene delivery vehicle followed by protein; multiple gene delivery vehicles followed by multiple protein administrations; protein administration(s) followed by single or multiple gene delivery vehicle administration; concurrent administration; and the like. Thus, a mixture of protein and nucleic acid can be administered, using the same or different vehicles and the same or different modes of administration.

In certain embodiments, direct delivery will generally be accomplished with or without viral vectors, as described above, by injection using either a conventional syringe or a gene gun, such as the Accell® gene delivery system (PowderJect Technologies, Inc., Oxford, England).

Thus, injection can be either subcutaneously, epidermally, intradermally, intramucosally such as nasally, rectally, orally and vaginally, intraperitoneally, intravenously, or intramuscularly. Other modes of administration include pulmonary administration, suppositories, needle-less injection, transcutaneous and transdermal applications. Dosage treatment may be a single dose schedule or a multiple dose schedule. As noted above, administration of nucleic acids may also be combined with administration of peptides or other substances.

### D. PHARMACEUTICAL COMPOSITIONS

The present disclosure also includes pharmaceutical compositions comprising a replication-defective vector (*e.g*., recombinant alphavirus construct or alphavirus particle) in combination with a pharmaceutically acceptable carrier, diluent, or recipient Further, other ingredients, such as adjuvants, may also be present. As described more fully in U.S. Patent No. 6,015,694, storage stable and easy administerable immunogenic compositions are particularly needed in Third World countries where refrigeration and/or traditional administration means (syringes, etc.) are not readily available.

In certain embodiments, polypeptides may also be included. The preparation of immunogenic compounds that contain immunogenic polypeptide(s) as active ingredients is known to those skilled in the art. Typically, such immunogenic compounds are prepared as injectables, either as liquid solutions or suspensions; solid forms suitable for solution in, or suspension in, liquid prior to injection can also be prepared. The preparation can also be emulsified, or the protein encapsulated in liposomes.

Composltions disclosed herein preferably comprise a pharmaceutically acceptable carrier. The carrier should not itself induce the production of antibodies harmful to the host. Pharmaceutically acceptable carriers are well known to those in the art. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Examples of particulate carriers include those derived from polymethyl methacrylate polymers, as well as microparticles derived from poly(lactides) and poly(lactide-co-glycolides), know as PLG. See, e.g., Jeffery et al., Pharm. Res. (1993) 10362-368; McGee et al. (1997) J Microencapsul. 14(2):197-210; O'Hagan et al. (1993) Vaccine 11(2):149-54. Such carriers are well known to those of ordinary skill in the art. Additionally, these carriers may function as immunostimulating agents ("adjuvants"). Furthermore, the antigen may be conjugated to a bacterial toxoid, such as toxoid from diphtheria, tetanus, cholera, etc., as well as toxins derived from *E*. *coli.*

Pharmaceutically acceptable salts can also be used in compositions disclosed herein for example, mineral salts such as hydrochlorides, hydrobromides, phosphates, or sulfates, as well as salts of organic acids such as acetates, proprionates, malonates, or benzoates. Especially useful protein substrates are serum albumins, keyhole limpet hemocyanin, immunoglobulin molecules, thyroglobulin, ovalbumin, tetanus toxoid, and other proteins well known to those of skill in the art. Compositions disclosed herein can also also contain liquids or excipients, such as water, saline, glycerol, dextrose, ethanol, or the like, singly or in combination, as well as substances such as wetting agents, emulsifying agents, or pH buffering agents. Liposomes can also be used as a carrier for a composition disclosed herein such liposomes are described above.

Briefly, with regard to viral particles, replication-defective vectors (also referred to above as particles) may be preserved either in crude or purified forms. Preservation methods and conditions are described in U.S. Patent No. 6,015,694.

Further, the compositions described herein can include various excipients, adjuvants, carriers, auxiliary substances, modulating agents, and the like. Preferably, the compositions will include an amount of the antigen sufficient to mount an immunological response. An appropriate effective amount can be determined by one of skill in the art. Such an amount will fall in a relatively broad range that can be determined through routine trials and will generally be an amount on the order of about 0.1 µg to about 1000 µg, more preferably about 1 µg to about 300 µg, of particle/antigen.

Such adjuvants include, but are not limited to: (1) aluminum salts (alum), such as aluminum hydroxide, aluminum phosphate, aluminum sulfate, etc.; (2) oil-in-water emulsion formulations (with or without other specific immunostimulating agents such as muramyl peptides (see below) or bacterial cell wall components), such as for example (a) MF59 (International Publication No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see.below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particle generated therefrom such as ISCOMs (immunostimulating complexes); (4) Complete Freunds Adjuvant (CFA) and Incomplete Freunds Adjuvant (IFA); (5) cytokines, such as interleukins (IL-1, IL-2, etc.), macrophage colony stimulating factor (M-CSF), tumor necrosis factor (TNF), beta chemokines (MIP, 1-alpha, 1-beta Rantes, etc.); (6) detoxified mutants of a bacterial ADP-ribosylating toxin such as a cholera toxin (CT), a pertussis toxin (PT), or an E. coli heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, e.g., International Publication Nos. WO93/13202; WO92/19265; WO 95/17211; WO 98/18928 and WO 01/22993); and (7) other substances that act as immunostimulating agents to enhance the effectiveness of the composition.

Muramyl peptides include, but are not limited to, N-acetyl-muramyl-L-threonyl-D-isoglutamine (thr-MDP), N-acteyl-normuramyl-L-alanyl-D-isogluatme (nor-MDP), N-acetylmuramyl-L-alanyl-D-isogluatminyl-L-alanine-2-(1'-2'-dipalmitoyl-sn-glycero-3-huydroxyphosphoryloxy)-ethylamine (MTP-PE), etc.

Administration of the pharmaceutical compositions described herein may be by any suitable route (see, *e.g*., Section C). Particularly preferred is mucosal (*e.g*., rectal and/or vaginal) administration. Dosage treatment may be a single dose schedule or a multiple dose schedule. A multiple dose schedule is one in which a primary course of vaccination may be with 1-10 separate doses, followed by other doses given at subsequent time intervals, chosen to maintain and/or reinforce the immune response, for example at 1-4 months for a second dose, and if needed, a subsequent dose(s) after several months. The dosage regimen will also, at least in part, be determined by the potency of the modality, the vaccine delivery employed, the need of the subject and be dependent on the judgment of the practitioner.

In yet other embodiments, a multiple administrations (*e.g*., prime-boost type administration) will be advantageously employed. For example, recombinant I alphavirus particles expressing the antigen(s) of interest are administered (*e.g*., IVAG or IR). Subsequently, the antigen(s) are administered, for example in compositions comprising the polypeptide antigen(s) and a suitable adjuvant. Alternatively, antigens are administered prior to gene delivery vehicles. Multiple polypeptide and multiple gene delivery vehicle administrations (in any order) may also be employed.

### E. METHODS FOR UTILIZING REPLICATION-DEFECTIVE PARTICLES AND ANTIGENIC POLYPEPTIDES

Within certain aspects of the present disclosure compositions and methods are provided for mucosally administering a composition (*e.g*., an alphavirus vector construct) that is capable of preventing, inhibiting, stabilizing or reversing sexually transmitted diseases. Representative examples of such diseases include bacterial and viral infections, particularly sexually transmitted infections, including but not limited to HIV, HBV HTLV I, HTLV II, HPV, HSV, HCV, chlamydia, gonorrhea, and/or syphilis.

More specifically, within one aspect of the present disclosure compositions and methods are provided for stimulating an immune response (either humoral or cell-mediated) to a sexually transmitted pathogenic agent, such that the pathogenic agent is either killed or inhibited. Representative examples of pathogenic agents include bacteria and viruses.

Within one embodiment of the disclosure the pathogenic agent is a virus, and methods are provided for stimulating a specific immune response and inhibiting viral spread by using recombinant alphavirus viral particles designed to deliver a vector construct that directs the expression of an antigen or modified form thereof to susceptible target cells capable of either (1) initiating an immune response to the viral antigen or (2) preventing the viral spread by occupying cellular receptors required for viral interactions. Expression of the vector nucleic acid encoded protein may be transient or stable with time. Where an immune response is to be stimulated to a pathogenic antigen, the recombinant alphavirus is preferably designed to express a modified form of the antigen which will stimulate an immune response and which has reduced pathogenicity relative to the native antigen. This immune response is achieved when cells present antigens in the correct manner, *i.e*., in the context of the MHC class I and/or II molecules along with accessory molecules such as CD3, ICAM-1, ICAM-2, LFA-1, or analogues thereof (*e.g*., Altmann et al., Nature 338:512, 1989). Cells infected with alphavirus vectors are expected to do this efficiently because they closely mimic genuine viral infection and because they: (a) are able to infect non-replicating cells, (b) do not integrate into the host cell genome, (c) are not associated with any life threatening diseases, and (d) express high levels ofheterologous protein. Because of these differences, alphavirus vectors can easily be thought of as safe viral vectors that can be used on healthy individuals for vaccine use.

This aspect of the disclosure has a further advantage over other systems that might be expected to function in a similar manner, in that the presenter cells are fully viable and healthy and low levels of viral antigens, relative to heterologous genes, are expressed. This presents a distinct advantage since the antigenic epitopes expressed can be altered by selective cloning of sub-fragments of the gene for the antigen into the recombinant alphavirus, leading to responses against immunogenic epitopes that may otherwise be overshadowed by immunodominant epitopes. Such an approach may be extended to the expression of a peptide having multiple epitopes, one or more of the epitopes being derived from different proteins. Further, this aspect of the invention allows efficient stimulation of cytotoxic T lymphocytes (CTL) directed against antigenic epitopes, and peptide fragments of antigens encoded by sub-fragments of genes, through intracellular synthesis and association of these peptide fragments with MHC Class I molecules. This approach may be utilized to map major immunodominant epitopes for CTL induction.

The compositions (*e.g*., alphavirus constructs and particles) suitable for mucosal administration described herein may include one or more inhibitory palliative, *e.g*., a viral or bacterial inhibitory gene which express anti-sense or the like. A discussion of inhibitory palliatives are described, for example, in U.S. Patent No. 6,015,686 which describes how antigens and inhibitory palliatives (*e.g*., expressing anti-sense tat, etc.) can be co-expressed and/or designed to overexpress proteins required for infection, such as CD4. In this way, a relatively small number of vector-infected HIV-resistant cells act as a "sink" or "magnet" for multiple nonproductive fusion events with free virus or virally infected cells. In the case of HIV, the two agents of interaction are the gp 120/gp 41 envelope protein and the CD4 receptor molecule. Thus, an appropriate blocker would be a vector construct expressing either an HIV env analogue that blocks HIV entry without causing pathogenic effects, or a CD4 receptor analogue. The CD4 analogue would be secreted and would function to protect neighboring cells, while the gp 120/gp 41 is secreted or produced only intracellularly so as to protect only the vector-containing cell. It may be advantageous to add human immunoglobulin heavy chains or other components to CD4 in order to enhance stability or complement lysis. Delivery of an alphavirus vector encoding such a hybrid-soluble CD4 to a host results in a continuous supply of a stable hybrid molecule. Efficacy of treatment can be assayed by measuring the usual indicators of disease progression, including antibody level, viral antigen production, infectious HIV levels, or levels of nonspecific infections.

Such a transcriptional repressor protein may be selected for in tissue culture using any viral-specific transcriptional promoter whose expression is stimulated by a virus-specific transactivating protein (as described above). In the specific case of HIV, a cell line expressing HIV tat protein and the HSVTK gene driven by the HIV promoter will die in the presence of ACV. However, if a series of mutated tat genes are introduced to the system, a mutant with the appropriate properties (*i.e*., represses transcription from the HIV promoter in the presence of wild-type tat) will grow and be selected. The mutant gene can then be reisolated from these cells. A cell line containing multiple copies of the conditionally lethal vector/tat system may be used to assure that surviving cell clones are not caused by endogenous mutations in these genes. A battery of randomly mutagenized tat genes is then introduced into these cells using a "rescuable" alphavirus vector (*i.e*., one that expresses the mutant tat protein and contains a bacterial origin of replication and drug resistance marker for growth and selection in bacteria). This allows a large number of random mutations to be evaluated and permits facile subsequent molecular cloning of the desired mutant cell line. This procedure may be used to identify and utilize mutations in a variety of viral transcriptional activator/viral promoter systems for potential antiviral therapies.

Additional inhibitory palliatives that may be used in the gene delivery vehicles described herein include systems wherein expression of the heterologous transgene may be reduced (suppressed) in desired cells, including during the virion packaging process, for example by including a TOP-binding ligand and packaging elements in the vector. As described, for example, in co-owned U.S. Serial Number 09/608,730, these methods allow for the suppression of transgene translation in virion producing cells while maintaining the capacity for high level expression and translation of the transgene in all other cell types. These systems are also applicable to a variety of viral vectors and, in addition, can be combined with other virion production systems, including, for example, adenovirus virion construction in a cre-lox system.

The following examples are offered by way of illustration, and not by way of limitation. Aspects of the following examples which do not relate specifically to the claimed invention are included for comparison and illustration only.

### EXAMPLE 1

### MATERIALS AND METHODS

### Mice and Cell lines

Female Balb/c mice were purchased from Charles River Breeding Laboratories and were at the age of 6 to 8 weeks at the onset of the studies. The fibroblast cell line SvBalb (H-2^{d}) was used as target cells. This cell line expresses class I but not class II MHC molecules and is therefore directed against CD8⁺ but not CD4⁺ cells.

### Materials

p7g is an H-2K^{d} restricted HIV-1_{SP2}p24gag CTL epitope and is a synthetic 9 mer peptide: (aa, 199-AMQMLKETI-207).(21) This peptide was synthesized with free amine N termini and free acid C termini using Fmoc solid phase methods by Research Genetics (Huntsville AL) (see, e.g., Mathiowitz et al. Nature 386:410-414).

### Immunizations

Groups of 5 female Balb/C mice were used for each vaccine or immunization route and the tissues were pooled upon sacrifice. The data are presented as representative of 2-4 such data points with similar or identical results. All immunizations were performed 3-4 times at 2-3 week intervals. IN immunizations were performed with 2.5E10⁶ SIN particles in a volume of 25 µl suspended in PBS. IN immunizations were performed without anesthesia. IVAG and IR immunizations with 2.5E10⁶ in a volume of 12.5 µl were performed on anesthetized mice that were kept in dorsal recumbency for 20 minutes. IM immunizations were performed in thigh muscle with 2.5E10⁶ SIN particles in a volume of 50 µl The mice were sacrificed one week following the final immunization.

### Sera and Tissue Collection

Mice were bled through the retro orbital plexus one day prior to sacrifice and the sera separated for ELISA assays. Cervical lymph nodes (CLN), iliac lymph nodes (ILN), vaginal/uteral mucosal tissues (VUM) and spleens (SP) were harvested and pooled from 5 mice per group and single cell suspensions were used for a standard "Cr-release CTL assay (except VUM), a gag epitope specific ELISPOT to detect IFN-γ-secreting cells (IFNSC) or gag-p55 specific ELISPOT to detect antibody secreting cells (ASC).

### Preparation of single cell suspensions

Groups of 5 mice were immunized 3 times as described above through the IN, IM, IR or IVAG routes with 2-3 week intervals. One week following the final immunization SP, CLN and ILN from groups of 5 immunized mice each were harvested and pooled. SP, CLN and ILN tissues were teased through a nylon mesh with pore diameter 250 µm and washed three times in media (ELISPOT assay media: RPMI containing 10% FCS, antibiotics, Hepes and L-glutamin (complete RPMI); ⁵¹Cr-release assay media), counted and seeded into wells for ELISPOT or ⁵¹Cr-release assay. Single cell suspensions from VUM were prepared based on the method described by Holmgren (see, for example, Lycke and Holmgren (1986) Immunology 59:301-308) with modifications by removing the entire vagina, uterus and uteral horns from pools of 5 mice per group. The uteral horns were cut longitudinally and together with vaginal and uteral tissues were diced into 5 mm pieces. The tissue pieces were then washed three times in HBSS without Ca++ and Mg++ containing 10% FCS and 5 mM Hepes (complete HBSS). The pieces were then treated enzymatically under agitation at 37°C sequentially once with 1 mg/ml Collagenase/Dispase plus 0.5 mg/ml DNase in complete HBSS for 30 min. and twice with collagenase plus 0.5 mg/ml DNase in complete RPMI for 45 min. Following each enzymatic treatment released cells were recovered and washed twice with complete RPMI. The cell suspensions recovered from each enzymatic treatment were pooled and counted. This method routinely resulted in recovery of a minimum of 10⁷ viable mononuclear cells (MNC) per five mice.

### ⁵¹Cr-release CTL Assay

Single cell suspensions from the tissues were prepared as described above and were cultured in a 24-well dish at 5x10⁶ cells per well. Of these cells, 1X10⁶ were sensitized with synthetic p7g peptide (amino acids 194-213) at a concentration of 10 mM for 1 hour at 37°C and then washed and co-cultured with the remaining 4x10⁶ untreated cells. The cells were stimulated as a bulk culture in 2 ml of Splenocyte culture medium: RPMI 1640 with 100 mM L-glutamine (Gibco, Grand Island, New York, USA)/-Mem (Minimum Essential Medium Alpha Medium with L-glutamine, deoxyribonucleosides or ribonucleosides) (1:1) supplemented with 10% heat-inactivated fetal calf serum (Hyclone, Logan, Utah, USA), 100 U/ml penicillin, 100 g/ml streptomycin, 10 ml/L of 100 mM sodium pyruvate and 50 M 2-mercaptoethanol. In addition, 5% Rat T-Stim IL2 (Rat T-Stim; Collaborative Biomedical Products, Bedford, Massachusetts, USA) was used as a source of IL2 and was added to the culture media just before the cells were to be cultured.

After a stimulation period of 6 to 7 days, the cells were collected and used as effectors in a ⁵¹Chromium release assay. Approximately 10⁶ SV/Balb target cells were incubated in 200 µl of medium containing 50 Ci of ⁵¹Cr and with the correct peptide p7g, or a mismatched cell-target pair as the negative control at a concentration of 1 M for 60 min and washed. Effector cells were cultured with 5x10³ target cells at various effector to target ratios in 200 µl of culture medium in 96-well tissue culture plates (round or v-bottom) for 4 hr. The average cpm from duplicate wells was used to calculate percent specific release as presented here.

### ELISPOT assays

Single cell suspensions from pooled CLN and SP from 5 mice per group were added onto nitrocellulose or pvdf plates (Milipore) precoated with a monoclonal rat anti-mouse anti-IFN-γ (Pharmingen) and p55 and blocked with complete RPMI medium at pH 7.2, containing 10% fetal calf serum, 5mM Hepes, and antibiotics. For detection of total p55-specific antibody secreting cells (ASC), following overnight incubation of cells at 37°C, the plates were washed with PBS/0.05% Tween (P/T). Biotinylated goat anti-IgH+L (Southern Biotechnology Associates, Birmingham, Alabama) was added at 1:7000 dilution in PBS/1% normal goat serum and incubated at room temperature (RT) for 2 hours. The plates were washed with P/T and incubated for 1 hr. at 37°C with Avidin-peroxidase at 1:1000 dilution (Pharmingen). The plates were washed with P/T and the spots were visualized by adding DAB in Tris-HCl buffer for 30 minutes. The plates were washed with de-ionized water and air-dried. The spots were counted manually under low magnification from duplicate wells per group and per tissue. The results are presented as ASC per 10 million cells and are representative of at least two experiments with similar results.

For detection of IFN-γ secreting cells following overnight incubation of cells in the presence of gag-derived p7g peptide, or anti-CD3 (Pharmingen) and anti-CD28 (Pharmingen) as positive control for polyclonal T cell activation, or media only as negative control, the plates were washed and biotinylated anti-IFN-γ (Pharmingen) was added in PBS./0.1% BSA/0.02% Tween and incubated at R/T for 2 hours. The plates were washed with P/T and incubated for 1 hr. at 37°C with Avidin-peroxidase (Pharmingen) at 1:1000 dilution. The plates were washed with P/T and the spots were visualized by adding DAB in Tris-HCl buffer for 30 minutes. The plates were washed with de-ionized H2O and air-dried. The spots were counted with an in house developed automated ELISPOT reader using software from Alpha Innotech Corporation (San Leandro, CA).

### ELISA assays

HIV-1 p55 gag specific serum IgG titers were quantified by a standard ELISA assay. Briefly, ELISA plates (96 well U bottom by Nunc Maxisorp) were coated with p55 protein at 5 µg/well. After washing with 1X PBS + 0.03% Tween 20 (Sigma), the wells were blocked and samples were added in serial dilutions in an assay diluent made up of 1X PBS + 5% goat serum (Gibco Brl) + 0.03% Tween 20 (Sigma). A standard serum was included in each assay for quantitation purposes. The samples and standard sera were incubated at 37°C for one hour and washed with PBS/0.03% Tween. The samples were then incubated with a 1:40000 dilution of a goat anti-mouse IgG-HRP (Caltag) and developed with tetramethylbenzidine (TMB-Kirkegaard and Perry) for 15 minutes and then stopped with 2N HCl. The optical density of each well was measured using Titertek at 450 nm.

### EXAMPLE 2

### CTL RESPONSES FOLLOWING MUCOSAL VS. SYSTEMIC IMMUNIZATIONS WITH SIN-GAG BEFORE CHALLENGE WITH VACCINIA VIRUS (VV)-GAG

The ability of SIN-gag particles to induce local and systemic gag-specific CTL responses was assessed through various routes of mucosal or systemic immunization. Mice were immunized intranasally (IN) or intramuscularly (IM) with 2.5E10⁶ SIN-gag particles and intravaginally (IVAG) or intrarectally (IR) with 10⁷ SIN-gag particles and sacrificed 7 days following the final immunization. To determine systemic gag-specific CTL responses, single cell suspensions from CLN, ILN, VUM and SP were prepared and a gag-specific IFN-γ. ELISPOT assay was performed. Local gag-specific CTL responses in CLN following IN immunizations were observed. In contrast, no local CTL responses ILN or in VUM, following IVAG and IR immunizations were seen (Figure 1). Moreover, systemic gag-specific CTL responses in SP following IN and IM immunizations were found, but not following IVAG and IR immunizations, (Figure 2). To assess the lytic activity of the CTL responses that were detected as the number of IFN-γ secreting cells by the ELISPOT assay, a standard ⁵¹Cr-release assay on CLN and SP cells following IN immunizations with SIN-gag particles was performed. Local and systemic lytic CTL activity in CLN and SP following IN immunizations with SIN-gag (Figure 3) was observed. Thus, IN or IM, but not IVAG or IR, immunizations with SIN-gag induced local and systemic CTL responses.

### EXAMPLE 3

### CTL RESPONSES FOLLOWING MUCOSAL VS. SYSTEMIC IMMUNIZATIONS WITH SIN-GAG AFTER CHALLENGE WITH VV-GAG

Local and systemic CTL responses following mucosal and systemic immunizations and local challenge were determined. (see, also, Vajdy et al. (2001) Journal of Infectious Disease 184:1613). Mice were immunized IN or IM with 2.5E10⁶ SIN-gag particles and 1-3 weeks following the final immunization were challenged IR or IVAG with 10⁷⁻⁸ pfu of Vaccinia virus (VV) expressing HIV-1 p55-gag and sacrificed 5 days following the final immunization. The following controls were used: a group of naïve mice were challenged IVAG, a group of naïve mice were challenged IR, groups of mice were immunized IM, IN, IR or IVAG with SIN-gag and challenged IVAG with Vaccinia virus expressing HIV-1 envelope gp160 (VV-env).

Following IM or IN immunizations with SIN-gag and vaginal challenge with VV-gag a potent gag-specific CTL responses in local, CLN, and systemic, SP, lymphoid tissues was detected. Importantly, potent CTL responses were also detected in the distant mucosal effector site, VUM as well as in the distant inductive site, ILN as measured by the ELISPOT assay (Figure 4). None of the control groups demonstrated local or systemic CTL responses (Figure 5). Thus, systemic injection in thigh muscle as well as mucosal administration onto the nasal mucosa with SIN-gag followed by vaginal challenge with VV-gag induced potent CTL responses not only in the local inductive site of the nasal mucosa (CLN), but also in the distant inductive (ILN) and effector (VUM) sites of the vaginal mucosa. Moreover, both IN and IM immunizations with SIN-gag followed by vaginal challenge with VV-gag induced potent systemic CTL responses.

To determine local and systemic CTL responses following local immunization and local challenge, mice were immunized IVAG or IR with 10⁷ SIN-gag particles and challenged IVAG with 10⁷ pfu VV-gag. Following IR immunizations with SIN-gag and IVAG challenge with VV-gag, local as well as systemic CTL responses in ILN and SP respectively, were detected, as measured by the IFN-γ ELISPOT assay (Figure 5). In contrast, mice immunized IVAG with SIN-gag and challenged IVAG with VV-gag demonstrated strong CTL responses locally in ILN and VUM but low CTL responses systemically in SP (Figure 5). None of the control groups demonstrated any local or systemic CTL responses. To confirm the lytic activity of the CTL responses detected by the ELISPOT technique, a standard Cr-release assay was performed with ILN and SP isolated from mice immunized IR or IVAG with SIN-gag and challenged IVAG with VV-gag and similar results were obtained. Thus, vaginal and rectal immunizations with SIN-gag particles induce local CTL responses in the mucosal effector and inductive sites. Furthermore, rectal, but not vaginal, immunization with SIN-gag particles followed by IVAG challenge with VV-gag evoked strong CTL responses systemic lymphoid tissue.

### EXAMPLE 4

### PROTECTION FROM MUCOSAL CHALLENGE WITH VV-GAG

To determine whether the presence of local and systemic CTL responses correlated with mucosal protection from various challenge a gag-expressing vaccinia virus challenge model was used. Vaccinia has been shown to infect and replicate in overies following intra-rectal, and various parenteral routes of inoculation. Two or three weeks following IR, IVAG, IN or IM immunizations with SIN-gag all groups of mice were challenged IVAG with 10⁷ pfu of VV-gag. Five days following IVAG challenge with VV-gag, the mice were sacrificed and a pfu assay performed on their ovaries. The following controls were used: naive mice challenged IVAG, naive mice challenged IR, groups of mice immunized IM, IN, IR or IVAG with SIN-gag and challenged IVAG with Vaccinia virus expressing HIV-1 envelope gp160 (VV-env).

Mice immunized IVAG or IR with SIN-gag were protected against IVAG and IR challenge with VV-gag respectively, in that no pfu was detected in their ovaries (Figure 6). Moreover, mice immunized IN with SIN-gag and challenged IVAG with VV-gag were only partially protected (Figure 6). Importantly, mice immunized IM and challenged IVAG were not protected (Figure VI). The naïve mice that were challenged with VV-gag IR or IVAG had high numbers of pfu in their ovaries (Figure 6). Moreover, the mice that were immunized IM, IN, IR or IVAG with SIN-gag and challenged IVAG with VV-env also had high pfu titers in their ovaries, demonstrating that the protection was antigen-specific (Figure VI). Importantly, mice immunized IM with SIN-gag and challenged intra-peritoneally with VV-gag were partially protected demonstrating that although IM immunization does not protect against mucosal challenge it does offer some degree of protection against systemic challenge (data not shown). Thus, local/mucosal, but not distant/mucosal or systemic, immunization protected the mice against local/mucosal challenge.

### EXAMPLE 5

### HIV-1 GAG-SPECIFIC CD8+ T CELL RESPONSES IN THE VAGINAL MUCOSA AND PROTECTION FROM VAGINAL VIRAL CHALLENGE FOLLOWING LOCAL IMMUNIZATIONS WITH SINDBIS VIRUS-BASED REPLICON PARTICLES

Groups of 5 BALB/c mice were used for each vaccine or immunization route and the tissues were pooled upon sacrifice. Mice were immunized by various routes 3 times at 2 week intervals, rested for 2-3 weeks and then challenged intra-vaginally (IVAG) or intra-rectally (IR) with 10⁷ plaque-forming units (PFU) of VV-gag or VV-gp160 (Gardner et al. (2000) J Virol 74:11849-57. SIN-gag particles were prepared as described in Gardner et al. (2000), *supra.* Intra-nasal (IN) immunizations were performed without anesthesia with 2.5x10⁶ SIN-gag particles in a volume of 25 µl suspended in PBS. The SIN-gag particles and the VV-gag virus were applied on anesthetized mice IVAG or IR in a volume of 12.5 µl following which the mice were kept in dorsal recumbency for 20 minutes. Intra-muscular (IM) immunizations were performed in thigh muscle with 2.5x10⁶ SIN particles in a volume of 50 µl. The mice were sacrificed 5 days following VV challenge for tissue collection.

Cervical lymph nodes (CLN), ILN, vaginal/uteral mucosal tissues (VUM) and spleens (SP) were harvested and pooled from 5 mice per group. Single cell suspensions were used for an ELISPOT assay to detect IFN-γ-secreting cells (IFNSC) specific for the p7g peptide derived from HIV-1 gag. Five days following vaginal or rectal challenge with VV-gag SP, CLN and ILN from groups of 5 immunized mice each were harvested and pooled. SP, CLN and ILN tissues were teased through a nylon mesh with the pore diameter of 250 µm and washed three times in media (ELISPOT assay media: RPMI containing 10% FCS, antibiotics, HEPES and L-glutamin (complete RPMI) counted and seeded into wells for ELISPOT assay. Single cell suspensions were prepared from VUM based on the method described by Johansson et al. (1998) Infect. Immun 66:514-520. This method routinely resulted in recovery of a minimum of 10⁷, minimum 90% viable, mononuclear cells (MNC) per five mice.

The p7g peptide derived from HIV-1 gag has been shown to be recognized by CD8⁺ T cells (Doe & Walker (1996) AIDS 10:793-94). It was also demonstrated that peptide was only recognized by CD8⁺ T cells and not by CD4⁺ T cells, following p7g-specific IFN-γ intracellular cytokine staining of CD4⁺ and CD8⁺ cells after DNA immunizations. Single cell suspensions from pooled VUM, ILN, CLN or SP from 5 mice per group were prepared and adjusted to concentrations of 10⁷ to 3×10⁷ per ml. 100 µl from each cell preparation was added onto the first row of 96 well nitrocellulose or pvdf plates (Milipore) in duplicates and 2-fold serial dilutions were performed. Following overnight incubation at 37°C the plates were washed and biotinylated anti-IFN-γ (PharMingen) was added. The plates were then incubated at room temperature for 2 hours and washed. The plates were then added Avidin-peroxidase (PharMingen) and incubated for 30 min. at 37°C and washed. The plates were developed with aminoethyl carbazole solution (Sigma) for 30 min. Results from 3 independent experiments are presented as mean (±SD) of IFN- y secreting cells per 10 million mononuclear cells (MNC) from a minimum of 4 wells from pools of 5 mice per group in duplicates.

### A. Mucosal and systemic CD8+ T cell responses after mucosal or systemic immunizations with SIN-gag followed by vaginal challenge with Vaccinia virus-gag

Viral challenge following immunization not only enhances the immune response but also allows a correlation to be drawn between the immune response and protection. Therefore, mice were primed with SIN-gag through the IN, IM, IR or IVAG routes and then challenged with VV-gag. The mucosal and systemic CTL responses as well as protection from VV-gag replication in ovaries was measured. The mice immunized IN, IM and IVAG were challenged IVAG, while the mice that were immunized IR were challenged IR or IVAG. Results of mucosal and systemic CD8⁺ T cell responses in VUM, ILN and SP of mice immunized IN or IM and challenged IVAG as measured by the IFN-γ ELISPOT assay are shown in Figures 11A and 11B, respectively. No CD8⁺T cell responses were detectable in CLN of either group. These responses were gag-specific since IVAG or IR challenge of naive mice with VV-gag, or IVAG or IR challenge of SIN-gag immunized mice with VV-gp160 did not induce CD8⁺T cell responses. These data show that vaginal VV challenge of IN or IM immunized mice induced CD8⁺ T cell responses in VUM and ILN.

After IR immunization with SIN-gag followed by IVAG challenge with VV-gag the highest CD8⁺T cell responses were found in ILN and SP, and relatively low responses were found in VUM (Figure 11C). Similarly, after IVAG immunization with SIN-gag followed by IVAG challenge with VV-gag, the highest CD8⁺T cell responses were found in ILN and SP, and relatively low responses in VUM (Figure 11D). The CD8⁺ T cell responses observed after IVAG or IR immunizations and challenge were generally 10-fold lower compared to the CD8⁺ T cell responses observed after IN and IM immunizations followed by IVAG challenge.

### B. Protection from local viral replication after mucosal immunizations with SIN-gag followed by vaginal challenge with Vaccinia virus-gag

Protection from vaccinia virus replication in the ovaries of mice primed through various routes following IVAG or IR virus challenge, using a standard PFU assay was also tested. As shown in Figure 12, mice primed IM with SIN-gag were not protected from challenge with IVAG VV-gag. In addition, 5 of the 19 mice primed IN showed no evidence of VV replication in their ovaries. In contrast, following IVAG or IR challenge with VV-gag, mice primed IVAG or IR with SIN-gag were completely protected against IVAG (Figure 12) and IR challenge with VV-gag respectively, in that no vaccinia virus replication was detected in their ovaries. The control, naïve mice had high levels of VV in their ovaries after IR or IVAG challenge with VV-gag (Figure 12). Also, the control mice that were immunized IN, IM, IVAG and IR with SIN-gag and challenged IVAG with VV-gp160 had high levels of VV in their ovaries (Figure 12), demonstrating that the protection was gag-specific. Thus, local mucosal immunization, but not distant mucosal or systemic immunization, with SIN-gag replicon particles conferred maximum protection against vaginal viral challenge.

Thus, mucosal IVAG or IR immunization conferred protection against vaginal viral challenge. In addition, the data indicate that alphavirus based replicons provide an effective mechanism to induce such local protection. Comparison of the distant mucosal (IN), local mucosal (IVAG and IR) and systemic (IM) routes of immunization with SIN-gag replicon particles, followed by local mucosal challenge with VV-gag, demonstrated that the IN and IM immunization routes induced higher gag-specific mucosal CD8⁺ T cell mediated responses in the vaginal mucosa (VUM), the draining lymph nodes (ILN), and in SP. However, IVAG and IR immunizations conferred maximum protection against vaginal challenge with VV-gag. Furthermore, the responses were gag-specific since immunization with SIN-gag followed by vaginal challenge with VV-gp160 did not induce any gag-specific IFN-γ secreting CD8⁺ T cell responses, nor had any impact on VV replication in ovaries. IVAG challenge of naïve mice with VV-gag also failed to induce any detectable gag-specific IFN-γ CD8⁺ T cell responses. Therefore, the induction of IFN-γ CD8⁺ T cell responses was gag-specific and as a result of adaptive specific responses following SIN-gag immunization. VV-gag challenge was also not mediated by humoral responses as indicated by the fact that gag-specific serum titers (as measured by ELISA two weeks after mucosal immunizations with SIN-gag replicons) were undetectable anti-gag serum. Thus, the observed protection was a result of gag-specific T cell-mediated responses.

In summary, the results demonstrate that the SIN replicon delivery system can be applied mucosally for the induction of CD8⁺ T cell responses and protective immunity against HIV. These results have important implications for sexually transmitted pathogens in general as well as for therapeutic gene therapy against cervical, colon, and lung cancers.

### EXAMPLE 6

### IDENTIFICATION OF CELLS EXPRESSING HIV-1 GAG IN LOCAL AND SYSTEMIC LYMPHOID TISSUES

Cells that are involved in the uptake and expression of SIN-gag particles in local and systemic lymphoid tissues following mucosal immunization were identified by immunizing mice IN with a single dose of SIN-gag particles. Frozen sections from nasal associated lymphoid tissue (NALT), CLN and SP were stained with an antibody against gag. At 1 day post immunization many cells expressing gag both locally in NALT (Figure 9) and CLN (Figure 10) as well as systemically in SP (Figure 11) were found, although by far most cells appeared to be localized in SP. To identify the cells that express gag, cells were double strained with CD11b or CD11c as markers for monocyte lineage cells with potential APC activity, and B220 a maker for B cells. Most of the gag-expressing cells co-expressed CD11b, while only few co-expressed CD11c. Importantly, no B220⁺ cells co-expressed gag and the CD11b⁺ or CD11c⁺ gag-expressing cells were in extra-follicular T-cell areas. Thus, at early time points following in immunizations CD11b⁺ cells are the major cells with APC potential to express gag in both local and systemic lymphoid tissues.

### EXAMPLE 7

### HUMORAL RESPONSES IN SERA FOLLOWING INTRANASAL IMMUNIZATIONS

The ability of SIN replicons to induce antibody production was also determined.

### A. HIV-derived antigens

Two weeks following 3 IN immunizations with SIN replicons expressing gag, serum titers were measured by ELISA (Figure 12). Additionally, prime-boost experiments were conducted. First, following 3 IN immunizations with SIN-gag mice were boosted IN three times at 4 weeks intervals with p24 plus LTK63. Additionally, experiments were conducted in which animals were primed with SIN expressing HIV envelope gp140 and boosted with Ogp140 plus the mucosal adjuvant LTR72 and CpG. In these particular cases, low or no levels of antibody titers were observed (Figure 10), indicating that the protection observed following vaginal or rectal immunizations with gag was not antibody-mediated.

### B. Influenza-derived antigens

Two weeks following 1 or more mucosal (*e.g*., IN, IVAG, IR) immunizations with SIN replicons expressing influenza polypeptide(s) (HA), serum titers are measured by ELISA. Additionally, prime-boost experiments are conducted. First, following mucosal immunizations with SIN-HA mice are boosted mucosally one or more times with HA plus LTK63. Additionally, experiments are conducted in which animals were primed with SIN expressing HA antigens and boosted with HA polypeptides plus the mucosal adjuvant LTR72 and CpG. SIN-HA replicons will induce antibody production when administered mucosally.

### EXAMPLE 8

### INDUCTION OF IMMUNE RESPONSES FOLLOWING MUCOSAL DELIVERY OF ALPHAVIRUS REPLICON PARTICLE CHIMERAS

To demonstrate the ability of alphavirus replicon particle chimeras to induce antigen specific immune responses following mucosal delivery, intranasal immunization experiments similar to those described above were performed. Specifically, replicon particle chimeras between SIN and VEE, as described in U.S. Serial Number 60/295,451, were constructed such that SIN replicon RNA was packaged within VEE envelope glycoproteins (SIN/VEE) or VEE replicon RNA was packaged within SIN envelope glycoproteins (VEE/SIN). These replicon particles encoding HIV p55 gag antigen, as well as SIN replicon particles and VEE replicon particles also encoding the same HIV gag antigen were used to immunize mice intranasally.

The replicon particles were administered three times, at a dose of 2.5 x 10⁶ particles in 25 µl, with an immunization schedule of days 0,14, and 28. At two weeks following the final immunization, spleens were removed and gag-specific cellular responses were determined by IFN-γ ELISPOT assay. As shown in Figure 13, each of the replicon particle preparations induced gag-specific responses, with some variation in immunogenicity.

## Claims

1. A replication-defective alphavirus replicon particle containing an alphavirus vector construct comprising a heterologous polynucleotide encoding at least one first antigen or modified form thereof, said first antigen or modified form thereof being capable of stimulating an immune response in a subject when administered intranasally, wherein said replication-defective alphavirus replicon particle is a chimeric alphavirus particle containing a Venezuelan Equine Encephalitis (VEE) virus vector construct packaged with Sindbis (SIN) virus envelope glycoproteins, for use in a therapeutic method of treating a mammalian subject by generating an immune response in the subject by intranasal administration.

2. Use of a replication-defective alphavirus replicon particle containing an alphavirus vector construct comprising a heterologous polynucleotide encoding at least one first antigen or modified form thereof, said first antigen or modified form thereof being capable of stimulating an immune response in the subject when intranasally administered, wherein said replication-defective alphavirus replicon particle is a chimeric alphavirus particle containing a Venezuelan Equine Encephalitis (VEE) virus vector construct packaged with Sindbis (SIN) virus envelope glycoproteins, in the manufacture of a medicament for therapeutically treating a mammalian subject by generating an immune response in the subject by intranasal administration.

3. The alphavirus replicon particle of claim 1 or the use of claim 2, wherein the at least one antigen is derived from a sexually transmitted pathogen.

4. The alphavirus replicon particle or use of claim 3, wherein the sexually transmitted pathogen is a bacteria.

5. The alphavirus replicon particle or use of claim 4, wherein the bacteria is selected from the group consisting of gonorrhea, chlamydia and syphilis.

6. The alphavirus replicon particle or use of claim 3, wherein the sexually transmitted pathogen is a virus.

7. The alphavirus replicon particle or use of claim 6, wherein the virus is selected from the group consisting of HIV, HBV, HSV, HCV and HPV.

8. The alphavirus replicon particle or use of claim 7, wherein the virus is HIV-1.

9. The alphavirus replicon particle of claim 1 or the use of claim 2, wherein the at least one first antigen is an HIV Gag antigen.

10. The alphavirus replicon particle or use of claim 9, wherein the at least one first antigen comprises an H-2K^{d} restricted HIV-1 p24gag CTL epitope.

11. The alphavirus replicon particle or use of claim 10, wherein the H-2K^{d} restricted HIV-1 p24gag CTL epitope consists of the sequence AMQMLKETI.

12. The alphavirus replicon particle or use of any one of claims 1-3 or 6-11, for preventing, inhibiting, stabilizing or reversing HIV.

13. The alphavirus replicon particle of claim 1 or the use of claim 2, wherein the at least one first antigen is an influenza hemagglutinin (HA) antigen.

14. The alphavirus replicon particle or use of any of claims 1 to 13, wherein the subject is a mammal.

15. The alphavirus replicon particle or use of claim 14, wherein the mammal is a human.

16. The alphavirus replicon particle or use of any of claims 1-15, wherein the alphavirus vector is delivered to antigen presenting cells.

17. The alphavirus replicon particle or use of claim 16, wherein the antigen presenting cells are dendritic cells.

18. The alphavirus replicon particle or use of claim 17, wherein the dendritic cells are human.

19. The alphavirus replicon particle or use of any of claims 1 to 18, wherein the target cells are infected *in vivo.*

20. The alphavirus replicon particle or use of any of claims 1 to 19, wherein the antigen elicits an HLA class I-restricted immune response.

21. The alphavirus replicon particle or use of claim 20, wherein the antigen further elicits an HLA Class II-restricted immune response.

22. The alphavirus replicon particle or use of any of claims 1 to 21, including, prior or subsequent to the step of administering to target cells, introducing into target cells a nucleic acid molecule which encodes either Class I or Class II MHC protein, or combinations thereof, or a protein selected from the group consisting of CD3, ICAM-1, LFA-3 or analogues thereof.

23. The alphavirus replicon particle or use of any of claims 1 to 22, further comprising the step of administering at least one second gene delivery vehicle, said second gene delivery vehicle comprising polynucleotides encoding at least one second antigen or modified form thereof or an immunomodulatory factor.

24. The alphavirus replicon particle or use of claim 23, wherein the second gene delivery vehicle is administered mucosally.

25. The alphavirus replicon particle or use of claim 23, wherein the second gene delivery vehicle is administered non-mucosally.

26. The alphavirus replicon particle or use of any of claims 1 to 25, further comprising the step of administering one or polypeptides to the subject.

27. The alphavirus replicon particle or use of claim 26, wherein the polypeptides comprise at least one second antigen or modified form thereof.

28. The alphavirus replicon particle or use of claim 26, wherein the polypeptides comprise an immunomodulatory factor.

29. The alphavirus replicon particle or use of claim 26, wherein at least one of the polypeptides is administered mucosally.

30. The alphavirus replicon particle or use of claim 26, wherein at least one of the polypeptides is administered non-mucosally.

31. The alphavirus replicon particle or use of any one of claims 1 to 30, wherein the medicament is for administration with a detoxified mutant of a bacterial ADP-ribosylating toxin.

32. The alphavirus replicon particle or use of claim 31, wherein the detoxified mutant of a bacterial ADP-ribosylating toxin is selected from the group consisting of cholera toxin, pertussis toxin and *E. coli* heat-labile toxin.

## Patentansprüche

1. Replikationsdefektes Alphavirus-Replikonpartikel enthaltend ein Alphavirus-Vektorkonstrukt umfassend ein heterologes Polynukleotid, das mindestens ein erstes Antigen oder eine modifizierte Form davon kodiert, wobei das erste Antigen oder die modifizierte Form davon dazu in der Lage ist, bei einer intranasalen Verabreichung eine Immunantwort in einem Subjekt zu stimulieren, wobei das replikationsdefekte Alphavirus-Replikonpartikel ein chimäres Alphaviruspartikel ist, das ein in Hüllglykoproteine des Sindbis (SIN)-Virus verpacktes virales Vektorkonstrukt der Venezolanischen Pferdeenzephalitis (VEE) enthält, zur Verwendung in einem therapeutischen Verfahren zur Behandlung eines Säugersubjekts durch hervorrufen einer Immunantwort in dem Subjekt mittels intranasaler Verabreichung.

2. Verwendung eines replikationsdefekten Alphavirus-Replikonpartikels enthaltend ein Alphavirus-Vektorkonstrukt umfassend ein heterologes Polynukleotid, das mindestens ein erstes Antigen oder eine modifizierte Form davon kodiert, wobei das erste Antigen oder die modifizierte Form davon dazu in der Lage ist, bei einer intranasalen Verabreichung eine Immunantwort in einem Subjekt zu stimulieren, wobei das replikationsdefekte Alphavirus-Replikonpartikel ein chimäres Alphaviruspartikel ist, das ein in Hüllglykoproteine des Sindbis (SIN)-Virus verpacktes virales Vektorkonstrukt der Venezolanischen Pferdeenzephalitis (VEE) enthält, bei der Herstellung eines Medikaments zur therapeutischen Behandlung eines Säugersubjekts durch hervorrufen einer Immunantwort in dem Subjekt mittels intranasaler Verabreichung.

3. Alphavirus-Replikonpartikel nach Anspruch 1 oder Verwendung nach Anspruch 2, wobei das mindestens eine Antigen von einem sexuell übertragbaren Pathogen abgeleitet ist.

4. Alphavirus-Replikonpartikel oder Verwendung nach Anspruch 3, wobei das sexuell übertragbare Pathogen ein Bakterium ist.

5. Alphavirus-Replikonpartikel oder Verwendung nach Anspruch 4, wobei das Bakterium ausgewählt ist aus der Gruppe bestehend aus Gonorrhoebakterien, Chlamydien und Syphilisbakterien.

6. Alphavirus-Replikonpartikel oder Verwendung nach Anspruch 3, wobei das sexuell übertragbare Pathogen ein Virus ist.

7. Alphavirus-Replikonpartikel oder Verwendung nach Anspruch 6, wobei das Virus ausgewählt ist aus der Gruppe bestehend aus HIV, HBV, HSV, HCV und HPV.

8. Alphavirus-Replikonpartikel oder Verwendung nach Anspruch 7, wobei das Virus HIV-1 ist.

9. Alphavirus-Replikonpartikel nach Anspruch 1 oder Verwendung nach Anspruch 2, wobei das mindestens eine erste Antigen ein HIV-Gag-Antigen ist.

10. Alphavirus-Replikonpartikel oder Verwendung nach Anspruch 9, wobei das mindestens eine erste Antigen ein H-2K^{d}-beschränktes HIV-1-p24gag-CTL-Epitop umfasst.

11. Alphavirus-Replikonpartikel oder Verwendung nach Anspruch 10, wobei das H-2K^{d}-beschränkte HIV-1-p24gag-CTL-Epitop aus der Sequenz AMQMLKETI besteht.

12. Alphavirus-Replikonpartikel oder Verwendung nach einem der Ansprüche 1 bis 3 oder 6 bis 11, zur Prävention, Inhibition, Stabilisation oder Reversion von HIV.

13. Alphavirus-Replikonpartikel nach Anspruch 1 oder Verwendung nach Anspruch 2, wobei das mindestens eine erste Antigen ein Influenza-Hämagglutinin (HA)-Antigen ist.

14. Alphavirus-Replikonpartikel oder Verwendung nach einem der Ansprüche 1 bis 13, wobei das Subjekt ein Säuger ist.

15. Alphavirus-Replikonpartikel oder Verwendung nach Anspruch 14, wobei der Säuger ein Mensch ist.

16. Alphavirus-Replikonpartikel oder Verwendung nach einem der Ansprüche 1 bis 15, wobei der Alphavirusvektor an Antigen präsentierende Zellen verabreicht wird.

17. Alphavirus-Replikonpartikel oder Verwendung nach Anspruch 16, wobei die Antigen präsentierenden Zellen dendritische Zellen sind.

18. Alphavirus-Replikonpartikel oder Verwendung nach Anspruch 17, wobei die dendritischen Zellen humane Zellen sind.

19. Alphavirus-Replikonpartikel oder Verwendung nach einem der Ansprüche 1 bis 18, wobei die Zielzellen *in vivo* infiziert sind.

20. Alphavirus-Replikonpartikel oder Verwendung nach einem der Ansprüche 1 bis 19, wobei das Antigen eine HLA-Klasse I-beschränkte Immunantwort hervorruft.

21. Alphavirus-Replikonpartikel oder Verwendung nach Anspruch 20, wobei das Antigen des Weiteren eine HLA-Klasse II-beschränkte Immunantwort hervorruft.

22. Alphavirus-Replikonpartikel oder Verwendung nach einem der Ansprüche 1 bis 21, einschließend, vor oder nach dem Schritt der Verabreichung an Zielzellen, das Einführen eines Nukleinsäuremoleküls in die Zielzellen, das MHC-Protein entweder der Klasse I oder II oder Kombinationen davon oder ein Protein ausgewählt aus der Gruppe bestehend aus CD3, ICAM-1, LFA-3 oder Analoga davon kodiert.

23. Alphavirus-Replikonpartikel oder Verwendung nach einem der Ansprüche 1 bis 22, des Weiteren umfassend den Schritt der Verabreichung mindestens eines zweiten Gentransfervehikels, wobei das zweite Gentransfervehikel Polynukleotide umfasst, die mindestens ein zweites Antigen oder eine modifizierte Form davon oder einen immunmodulatorischen Faktor kodieren.

24. Alphavirus-Replikonpartikel oder Verwendung nach Anspruch 23, wobei das zweite Gentransfervehikel mukosal verabreicht wird.

25. Alphavirus-Replikonpartikel oder Verwendung nach Anspruch 23, wobei das zweite Gentransfervehikel nicht-mukosal verabreicht wird.

26. Alphavirus-Replikonpartikel oder Verwendung nach einem der Ansprüche 1 bis 25, des Weiteren umfassend den Schritt der Verabreichung eines oder Polypeptide an das Subjekt.

27. Alphavirus-Replikonpartikel oder Verwendung nach Anspruch 26, wobei die Polypeptide mindestens ein zweites Antigen oder eine modifizierte Form davon umfassen.

28. Alphavirus-Replikonpartikel oder Verwendung nach Anspruch 26, wobei die Polypeptide einen immunmodulatorischen Faktor umfassen.

29. Alphavirus-Replikonpartikel oder Verwendung nach Anspruch 26, wobei mindestens eines der Polypeptide mukosal verabreicht wird.

30. Alphavirus-Replikonpartikel oder Verwendung nach Anspruch 26, wobei mindestens eines der Polypeptide nicht-mukosal verabreicht wird.

31. Alphavirus-Replikonpartikel oder Verwendung nach einem der Ansprüche 1 bis 30, wobei das Medikament zur Verabreichung mit einer detoxifizierten Mutante eines bakteriellen ADP-ribosylierenden Toxins vorgesehen ist.

32. Alphavirus-Replikonpartikel oder Verwendung nach Anspruch 31, wobei die detoxifizierte Mutante eines bakteriellen ADP-ribosylierenden Toxins ausgewählt ist aus der Gruppe bestehend aus Choleratoxin, Pertussistoxin und hitzelabilem E. Coli-Toxin.

## Revendications

1. Particule de réplicon d'alphavirus à réplication défectueuse contenant une construction de vecteur d'alphavirus comprenant un polynucléotide hétérologue codant au moins un premier antigène ou une forme modifiée de celui-ci, ledit premier antigène ou ladite forme modifiée de celui-ci étant capable de stimuler une réponse immune chez un patient quand il est administré par voie intranasale, dans lequel ladite particule de réplicon d'alphavirus à réplication défectueuse étant une particule d'alphavirus chimérique contenant une construction vectorielle de virus d'encéphalomyélite équine du Venezuela (VEE) encapsulée avec des glycoprotéines d'enveloppe virale Sindbis (SIN), pour une utilisation dans un procédé thérapeutique de traitement d'un mammifère en générant une réponse immune chez le patient par administration intranasale.

2. Utilisation d'une particule de réplicon d'alphavirus à réplication défectueuse contenant une construction de vecteur d'alphavirus comprenant un polynucléotide hétérologue codant au moins un premier antigène ou une forme modifiée de celui-ci, ledit premier antigène ou une forme modifiée de celui-ci étant capable de stimuler une réponse immune chez le patient quand il est administré par voie intranasale, dans lequel ladite particule de réplicon d'alphavirus à réplication défectueuse est une particule d'alphavirus chimérique contenant une construction vectorielle de virus d'encéphalomyélite équine du Venezuela (VEE) encapsulée avec des glycoprotéines d'enveloppe virale Sindbis (SIN), dans la fabrication d'un médicament pour traiter thérapeutiquement un mammifère en générant une réponse immune chez le patient par administration intranasale.

3. Particule de réplicon d'alphavirus selon la revendication 1 ou utilisation selon la revendication 2, dans laquelle le au moins un antigène est obtenu à partir d'un pathogène transmis sexuellement.

4. Particule de réplicon d'alphavirus ou utilisation selon la revendication 3, dans laquelle le pathogène transmis sexuellement est une bactérie.

5. Particule de réplicon d'alphavirus ou utilisation selon la revendication 4, dans laquelle la bactérie est sélectionnée parmi le groupe composé de gonorrhée, chlamydia et syphilis.

6. Particule de réplicon d'alphavirus ou utilisation selon la revendication 3, dans laquelle le pathogène transmis sexuellement est un virus.

7. Particule de réplicon d'alphavirus ou utilisation selon la revendication 6, dans laquelle le virus est sélectionné parmi le groupe composé de HIV, HBV, HSV, HCV et HPV.

8. Particule de réplicon d'alphavirus ou utilisation selon la revendication 7, dans laquelle le virus est l'HIV-1.

9. Particule de réplicon d'alphavirus selon la revendication 1 ou utilisation selon la revendication 2, dans laquelle le au moins un premier antigène est un antigène HIV Gag.

10. Particule de réplicon d'alphavirus ou utilisation selon la revendication 9, dans laquelle le au moins un premier antigène comprend un épitope CTL HIV-1 p24gag restreint au H-2Kd.

11. Particule de réplicon d'alphavirus ou utilisation selon la revendication 10, dans laquelle le épitope CTL HIV-1 p24gag restreint au H-2Kd est composé de la séquence AMQMLKETI.

12. Particule de réplicon d'alphavirus ou utilisation selon l'une quelconque des revendications 1 à 3 ou 6 à 11, pour empêcher, inhiber, stabiliser ou inverser le HIV.

13. Particule de réplicon d'alphavirus selon la revendication 1 ou utilisation selon la revendication 2, dans laquelle le au moins un premier antigène est un antigène d'influenza hémagglutinine (HA).

14. Particule de réplicon d'alphavirus ou utilisation selon l'une quelconque des revendications 1 à 13, dans laquelle le patient est un mammifère.

15. Particule de réplicon d'alphavirus ou utilisation selon la revendication 14, dans laquelle le mammifère est un être humain.

16. Particule de réplicon d'alphavirus ou utilisation selon l'une quelconque des revendications 1 à 15, dans laquelle le vecteur d'alphavirus est délivré aux cellules présentant un antigène.

17. Particule de réplicon d'alphavirus ou utilisation selon la revendication 16, dans laquelle les cellules présentant un antigène sont des cellules dendritiques.

18. Particule de réplicon d'alphavirus ou utilisation selon la revendication 17, dans laquelle les cellules dendritiques sont humaines.

19. Particule de réplicon d'alphavirus ou utilisation selon l'une quelconque des revendications 1 à 18, dans laquelle les cellules cibles sont infectées in vivo.

20. Particule de réplicon d'alphavirus ou utilisation selon l'une quelconque des revendications 1 à 19, dans laquelle l'antigène provoque une réponse immune restreinte à la HLA de classe I.

21. Particule de réplicon d'alphavirus ou utilisation selon la revendication 20, dans laquelle l'antigène provoque également une réponse immune restreinte à la HLA de classe II.

22. Particule de réplicon d'alphavirus ou utilisation selon l'une quelconque des revendications 1 à 21, comprenant, avant ou à la suite de l'étape d'administration aux cellules cibles, l'introduction dans les cellules cibles d'une molécule d'acide nucléique codant une protéine MHC de Classe I ou de Classe II, ou des combinaisons de celle-ci, ou une protéine sélectionnée parmi le groupe composé de CD3, ICAM-1, LFA-3 ou analogues de celui-ci.

23. Particule de réplicon d'alphavirus ou utilisation selon l'une quelconque des revendications 1 à 22, comprenant également l'étape d'administration d'au moins un second véhicule de délivrance de gène, ledit second véhicule de délivrance de gène comprenant des polynucléotides codant au moins un second antigène ou une forme modifiée de celui-ci ou un facteur immunomodulatoire.

24. Particule de réplicon d'alphavirus ou utilisation selon la revendication 23, dans laquelle le second véhicule de délivrance de gène est administré par voie mucosale.

25. Particule de réplicon d'alphavirus ou utilisation selon la revendication 23, dans laquelle le second véhicule de délivrance de gène est administré par voie non mucosale.

26. Particule de réplicon d'alphavirus ou utilisation selon l'une quelconque des revendications 1 à 25, comprenant également l'étape d'administration d'un ou de plusieurs polypeptides au patient.

27. Particule de réplicon d'alphavirus ou utilisation selon la revendication 26, dans laquelle les polypeptides comprennent au moins un second antigène ou une forme modifiée de celui-ci.

28. Particule de réplicon d'alphavirus ou utilisation selon la revendication 26, dans laquelle les polypeptides comprennent un facteur immunomodulatoire.

29. Particule de réplicon d'alphavirus ou utilisation selon la revendication 26, dans laquelle au moins l'un des polypeptides est administré par voie mucosale.

30. Particule de réplicon d'alphavirus ou utilisation selon la revendication 26, dans laquelle au moins l'un des polypeptides est administré par voie non mucosale.

31. Particule de réplicon d'alphavirus ou utilisation selon l'une quelconque des revendications 1 à 30, dans laquelle le médicament est destiné à une administration avec un mutant détoxifié d'une toxine bactérienne d'ADP-ribosylation.

32. Particule de réplicon d'alphavirus ou utilisation selon la revendication 31, dans laquelle le mutant détoxifié d'une toxine bactérienne d'ADP-ribosylation est sélectionnée parmi le groupe composé de toxine du choléra, toxine de la coqueluche et toxine E. coli instable à la chaleur.
